(19)

(11) **EP 3 909 552 B1**

(12) **FASCICULE DE BREVET EUROPEEN**

(45) Date de publication et mention de la délivrance du brevet:
**08.03.2023 Bulletin 2023/10**

(21) Numéro de dépôt: **21180949.6**

(22) Date de dépôt: **06.04.2017**

(51) Classification Internationale des Brevets (IPC):
***A61F 9/008*** *(2006.01)*

(52) Classification Coopérative des Brevets (CPC):
**A61F 9/00825;** A61F 2009/0087; A61F 2009/00872; A61F 2009/00897

(54) **SYSTÈME OPTIQUE DE FOCALISATION D'UN APPAREIL DE DÉCOUPE INCLUANT UN MODULATEUR SPATIAL DE LUMIÈRE**

OPTISCHES FOKUSSIERUNGSSYSTEM FÜR EIN SCHNEIDEGERÄT, DAS EINEN RÄUMLICHEN LICHTMODULATOR UMFASST

OPTICAL FOCUSING SYSTEM OF A CUTTING APPARATUS INCLUDING A LIGHT SPATIAL MODULATOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.04.2016 FR 1653038**
**06.04.2016 FR 1653039**
**06.04.2016 FR 1653040**
**29.07.2016 FR 1657386**

(43) Date de publication de la demande:
**17.11.2021 Bulletin 2021/46**

(62) Numéro(s) de document de la (des) demande(s) initiale(s) en application de l'article 76 CBE:
**17714828.5 / 3 439 593**

(73) Titulaires:
- **Keranova**
  **42000 Saint Etienne (FR)**
- **Université Jean Monnet Saint Etienne**
  **42023 Saint Etienne Cedex 2 (FR)**
- **Centre National de la Recherche Scientifique**
  **75794 Paris Cedex 16 (FR)**

(72) Inventeurs:
- **ROMANO, Fabrizio**
  **01700 Beynost (FR)**
- **BERNARD, Aurélien**
  **42000 Saint Etienne (FR)**
- **MAUCLAIR, Cyril**
  **49440 Challain La Potherie (FR)**
- **BAUBEAU, Emmanuel**
  **42000 Saint Etienne (FR)**

(74) Mandataire: **Be IP**
**Cabinet LTL SAS**
**Centre d'Entreprise et d'Innovation**
**56, Bd Niels Bohr**
**CS 52132**
**69603 Villeurbanne Cedex (FR)**

(56) Documents cités:
**EP-A1- 1 790 383**
**WO-A1-02/094117**
**WO-A1-2016/055539**
**US-A1- 2010 133 246**
**US-A1- 2016 067 095**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**EP 3 909 552 B1**

## Description

### DOMAINE TECHNIQUE

La présente invention concerne le domaine technique des opérations chirurgicales réalisées au laser femtoseconde, et plus particulièrement celui de la chirurgie ophtalmologique pour notamment des applications de découpes de cornées, ou de cristallins.

L'invention concerne un dispositif de découpe d'un tissu humain ou animal, telle qu'une cornée, ou un cristallin, au moyen d'un laser femtoseconde.

Par laser femtoseconde, on entend une source lumineuse, apte à émettre un faisceau L.A.S.E.R. sous forme d'impulsions ultra-courtes, dont la durée est comprise entre 1 femtoseconde et 100 picosecondes, de préférence comprise entre 1 et 1000 femtosecondes, notamment de l'ordre de la centaine de femtosecondes.

### ART ANTERIEUR

Il est connu de l'état de la technique de réaliser des opérations chirurgicales de l'oeil au moyen d'un laser femtoseconde, telles que des opérations de découpes de cornées ou de cristallins.

Le laser femtoseconde est un instrument apte à réaliser une découpe du tissu cornéen par exemple, en focalisant un faisceau L.A.S.E.R. dans le stroma de la cornée, et en réalisant une succession de petites bulles de cavitation adjacentes, qui forme ensuite une ligne de découpe.

Plus précisément, lors de la focalisation du faisceau L.A.S.E.R. dans la cornée, un plasma est généré par ionisation non-linéaire lorsque l'intensité du laser dépasse une valeur seuil, nommée seuil de claquage optique. Une bulle de cavitation se forme alors, engendrant une disruption très localisée des tissus environnant. Ainsi, le volume réellement ablaté par le laser est très faible comparativement à la zone disruptée.

La zone découpée par le laser à chaque impulsion est très petite, de l'ordre du micron ou de la dizaine de micron selon la puissance et la focalisation du faisceau. Ainsi, une découpe lamellaire cornéenne ne peut être obtenue qu'en réalisant une série d'impacts contigus sur toute la surface de la zone à découper.

Le déplacement du faisceau peut alors être réalisé par un dispositif de balayage, composé de miroirs galvanométriques pilotables, et/ou de platines permettant le déplacement d'éléments optiques, tels que des miroirs ou des lentilles. Ce dispositif de balayage permet de déplacer le faisceau suivant une trajectoire en va-et-vient le long d'une succession de segments formant un chemin de déplacement du faisceau.

Pour découper une cornée sur une surface de 1mm$^2$, il faut réaliser environ 20000 impacts très proches les uns des autres. Aujourd'hui ces impacts sont réalisés un par un à une vitesse moyenne de 300000 impacts/seconde. Pour découper une cornée sur une surface d'environ 65mm$^2$, en tenant compte des temps pendant lesquels le laser arrête la production des impulsions en bout de segment pour permettre aux miroirs de se positionner sur le segment suivant, il faut en moyenne 15 secondes. L'opération chirurgicale de découpe est donc lente.

Pour optimiser le temps de découpe, il est connu d'augmenter la fréquence du laser. Cependant, l'augmentation de la fréquence implique également une augmentation de la vitesse de déplacement du faisceau, au moyen de platines ou de scanners adaptés. Il est également connu d'augmenter l'espacement entre les impacts du laser sur le tissu à découper, mais généralement au détriment de la qualité de la découpe.

La plupart des lasers femtosecondes pour la découpe cornéenne utilisent ainsi de hautes fréquences de travail, notamment supérieures à 100kHz, associées à des systèmes de déplacement du faisceau combinant des scanners et des platines de déplacement, ce qui grève le coût total de l'installation, et donc de l'opération chirurgicale facturée.

Pour remédier à ce problème de rapidité de la découpe L.A.S.E.R., il est aussi connu d'utiliser des miroirs galvanométriques pour augmenter la cadence, la vitesse, et le trajet de déflection du faisceau L.A.S.E.R.

Cependant, cette technique ne donne pas entière satisfaction en termes de résultats.

Une autre solution pour diminuer le temps de découpe consiste à générer plusieurs bulles de cavitation simultanément. Les documents US 2010/133246, EP 1790 383 et US 2016/067095 décrivent des dispositifs de découpe basés sur la technique de subdivision d'un faisceau L.A.S.E.R. primaire unique en une pluralité de faisceaux L.A.S.E.R. secondaires. Ces dispositifs comprennent généralement un système optique - tel qu'un (ou plusieurs) séparateur(s) de faisceau - pour produire des faisceaux L.A.S.E.R. secondaires permettant de générer chacun une bulle de cavitation respective.

Le fait de générer simultanément « *n* » bulles de cavitation permet de diminuer la durée totale de la découpe d'un facteur « n ». La démultiplication d'un faisceau en plusieurs faisceaux, dans le but d'accélérer la procédure, a été déjà décrite mais toujours au moyen de solutions purement optiques, soit par diffraction, soit par réflexions multiples. Le résultat n'a jamais été exploitable en clinique, principalement car les différents faisceaux n'étaient pas de taille homogène.

Par ailleurs, la technique de subdivision induit une augmentation du diamètre de la pluralité de faisceaux L.A.S.E.R. secondaires par rapport au diamètre du faisceau L.A.S.E.R. primaire unique produit par le laser femtoseconde. En effet, les faisceaux L.A.S.E.R. secondaires correspondent à des « portions » du faisceau L.A.S.E.R. primaire unique séparées spatialement. Du fait de la distance non-nulle entre les différents faisceaux L.A.S.E.R. secondaires, le diamètre de l'ensemble que forment la pluralité de faisceaux L.A.S.E.R. secondaires est supérieur au diamètre du faisceau L.A.S.E.R. primaire.

Cette augmentation de diamètre peut être un inconvénient, notamment dans le cas où le dispositif de découpe comprend un système de balayage - tel qu'un scanner optique - pour déplacer la pluralité de faisceaux L.A.S.E.R. secondaires dans un plan de découpe. En effet, le diamètre d'entrée d'un système de balayage est généralement de l'ordre du diamètre du faisceau L.A.S.E.R. primaire unique de sorte que certains faisceaux secondaires ne pénètrent pas dans le système de balayage.

Ainsi à l'heure actuelle, les solutions de découpe à base de faisceau L.A.S.E.R. sont uniquement utilisées pour générer des bulles de cavitation le long d'une ligne de découpe, voir dans certains cas pour générer des bulles de cavitation dans un plan de découpe.

Toutefois, aucune des solutions existantes ne permet de détruire un volume de tissu, notamment du fait que l'empilement de plans de découpe est trop fastidieux à mettre en oeuvre et que la durée associée à un tel empilement de plan est incompatible avec la durée d'une intervention chirurgicale.

Un but de la présente invention est de proposer un appareil de découpe permettant de pallier au moins l'un des inconvénients précités. Notamment, un but de la présente invention est de proposer un appareil de découpe permettant de détruire un volume de tissu à traiter de manière rapide et efficace.

## EXPOSE DE L'INVENTION

A cet effet l'invention propose un appareil de découpe d'un tissu humain ou animal, telle qu'une cornée, ou un cristallin, ledit appareil incluant un laser femtoseconde apte à émettre un faisceau L.A.S.E.R. sous forme d'impulsions et un dispositif de traitement pour produire un motif dans un plan de focalisation à partir du faisceau L.A.S.E.R. généré par le laser femtoseconde, le dispositif de traitement étant disposé en aval dudit laser femtoseconde, *remarquable en ce que* le dispositif de traitement comprend :

- un modulateur spatial de lumière entre la source et le plan de focalisation, ledit modulateur spatial de lumière étant programmé à l'aide d'au moins un masque de phase pour moduler la phase du front d'onde du faisceau L.A.S.E.R. issu du laser femtoseconde de sorte à obtenir un faisceau L.A.S.E.R. modulé,
- un système optique de focalisation pour focaliser le faisceau L.A.S.E.R. modulé dans un plan de découpe, et
- une unité de commande apte à piloter le déplacement du système optique de focalisation le long d'un chemin optique du faisceau L.A.S.E.R. pour déplacer le plan de focalisation en au moins trois plans de découpe respectifs de sorte à former un empilement de surfaces de découpe du tissu.

Des aspects préférés mais non limitatifs de l'appareil de découpe sont les suivants :

- l'unité de de commande peut être apte à piloter le déplacement du système optique de focalisation pour déplacer le plan de focalisation entre une position initiale et une position finale dans cet ordre, la position finale étant plus proche du laser femtoseconde que la position initiale ;
- chaque masque de phase peut être une image bidimensionnelle dont chaque point est associé à un pixel respectif du modulateur spatial de lumière, ladite image bidimensionnelle déterminant comment la phase du faisceau doit être modifiée pour obtenir une répartition d'amplitude donnée dans le plan de focalisation ;
- le modulateur spatial de lumière peut être positionné entre le laser femtoseconde et le système optique de focalisation, au moins un masque de phase étant calculé pour répartir l'énergie du faisceau L.A.S.E.R. modulé en au moins deux points d'impact formant le motif dans le plan de focalisation ;
- ledit et au moins un masque de phase peut être calculé en utilisant un algorithme itératif basé sur la transformée de Fourier de sorte à obtenir un unique faisceau L.A.S.E.R. modulé en phase ;
- le modulateur spatial de lumière (SLM) peut être positionné entre le laser femtoseconde et le système optique de focalisation, au moins un masque de phase étant calculé pour modifier la forme géométrique d'un point d'impact du faisceau L.A.S.E.R. modulé, de sorte que :
    - la forme géométrique, dans le plan de focalisation, d'un point d'impact du faisceau L.A.S.E.R. issu du laser femtoseconde est différente de
    - la forme géométrique, dans le plan de focalisation, d'un point d'impact du faisceau L.A.S.E.R. modulé ;
- l'unité de commande peut être programmée pour piloter le modulateur spatial de lumière de sorte à faire varier la forme du motif entre deux plans de découpe respectifs ;

- l'unité de commande peut être programmée pour piloter le modulateur spatial de lumière, ladite unité de commande étant adaptée pour émettre au moins des premier et deuxième signaux de commande entre deux plans de découpe respectifs :

    - le premier signal de commande induisant la modulation de la phase du front d'onde du faisceau L.A.S.E.R. selon un premier masque de phase calculé pour répartir l'énergie du faisceau L.A.S.E.R. modulé en une pluralité de premiers points d'impact dans le plan de focalisation, les premiers points d'impact constituant un premier motif,
    - le deuxième signal de commande induisant la modulation de la phase du front d'onde du faisceau L.A.S.E.R. selon un deuxième masque de phase calculé pour répartir l'énergie du faisceau L.A.S.E.R. modulé en une pluralité de deuxièmes points d'impact dans le plan de focalisation, les deuxièmes points d'impact constituant un deuxième motif différent du premier motif ;

- l'appareil peut comprendre en outre un scanner optique de balayage disposé en aval du laser femtoseconde, pour déplacer le motif dans le plan de découpe en une pluralité de positions selon une direction de déplacement ;
- l'unité de commande peut être programmée pour piloter le scanner optique de balayage de sorte à faire varier l'aire découpée dans le plan de focalisation entre deux plans de découpe successifs ;
- l'unité de commande peut être programmée pour piloter le scanner optique de balayage de sorte à faire varier la forme de la zone découpée dans le plan de focalisation entre deux plans de découpe successifs ;
- l'unité de commande peut être programmée pour piloter le scanner optique de sorte à faire varier un pas de balayage du scanner optique entre deux plans de découpe successifs ;
- l'unité de commande peut être apte à piloter le déplacement du système optique de focalisation de sorte que la distance entre deux plans successifs soit comprise entre 2 μm et 500 μm ;
- l'appareil peut comprendre en outre un filtre disposé en aval du modulateur spatial de lumière (SLM) pour bloquer une énergie parasite générée au centre du modulateur spatial de lumière (SLM) ;
- le filtre peut comprendre une plaque incluant :

    - une zone opaque au rayonnement L.A.S.E.R. disposée au centre de la plaque, et
    - une zone transparente au rayonnement L.A.S.E.R. s'étendant à la périphérie de la zone opaque.

## BREVE DESCRIPTION DES DESSINS

D'autres caractéristiques et avantages de l'invention ressortiront clairement de la description qui en est réalisée ci-après, à titre indicatif et nullement limitatif, en référence aux figures annexées, dans lesquelles :

- la figure 1 est une représentation schématique d'un montage incluant l'appareil de découpe selon l'invention ;
- la figure 2 illustre une répartition d'intensité d'un faisceau L.A.S.E.R. dans son plan focal ;

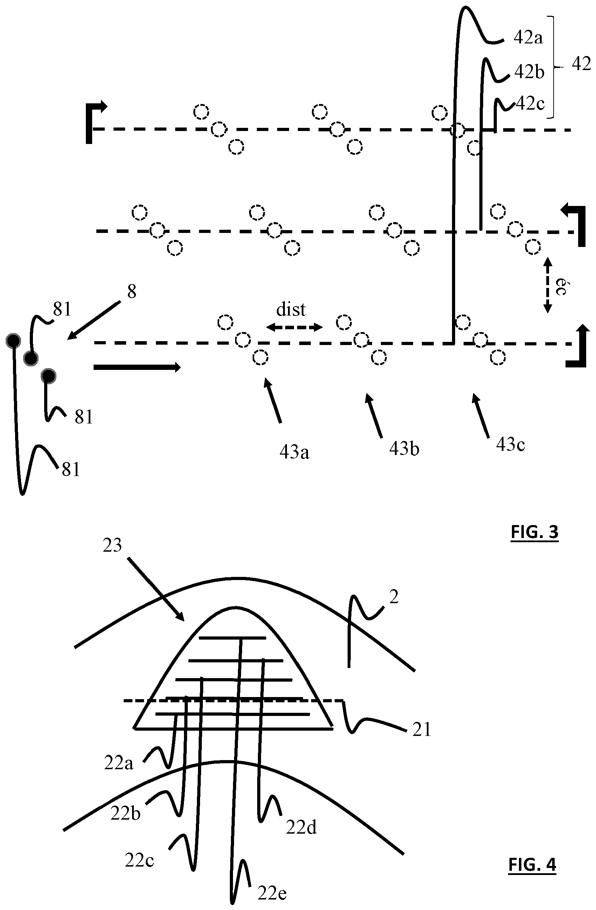

- la figure 3 illustre un chemin de déplacement d'un motif de découpe ;
- la figure 4 illustre des plans de découpe d'un volume de tissu à détruire ;
- les figures 5 à 9, 11 à 18, et 20 à 22, 24 et 28 illustrent différents exemples de motif de découpe,

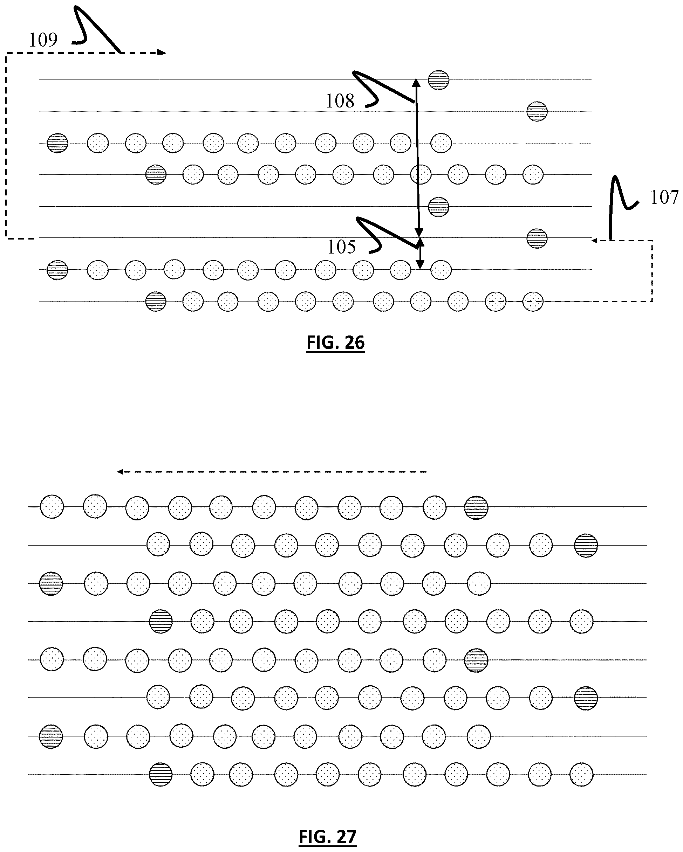

- les figures 10, 19, 23 et 25 à 27 illustrent des matrices de bulles de cavitation.

## EXPOSE DETAILLE DE L'INVENTION

L'invention concerne un appareil de découpe d'un tissu humain au moyen d'un laser femtoseconde. Dans la suite de la description, l'invention sera décrite, à titre d'exemple, pour la découpe d'une cornée d'un oeil humain ou animal.

### *1. Appareil de découpe*

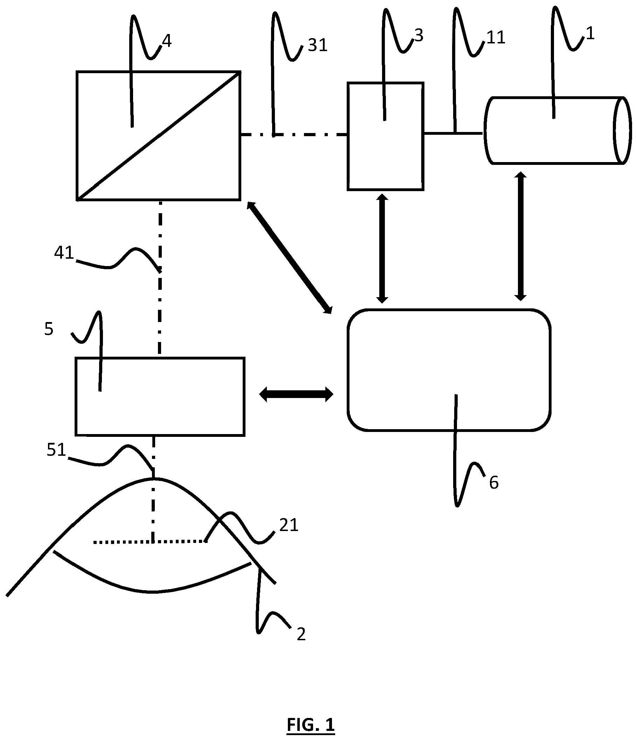

En référence à la figure 1, on a illustré un mode de réalisation de l'appareil de découpe selon l'invention. Celuici peut être disposé entre un laser femtoseconde 1 et une cible à traiter 2.

Le laser femtoseconde 1 est apte à émettre un faisceau L.A.S.E.R. sous la forme d'impulsions. A titre d'exemple, le laser 1 émet une lumière de 1030 nm de longueur d'onde, sous la forme d'impulsions de 400 femtosecondes. Le laser 1 possède une puissance de 20W et une fréquence de 500 kHz.

La cible 2 est par exemple un tissu humain ou animal à découper tel qu'une cornée ou un cristallin.

L'appareil de découpe comprend :

- un système de mise en forme 3 positionné sur la trajectoire du faisceau L.A.S.E.R. 11 issu du laser femtoseconde 1,

- un scanner optique de balayage 4 en aval du système de mise en forme 3,
- un système optique de focalisation 5 en aval du scanner optique de balayage 4.

L'appareil de découpe comprend également une unité de commande 6 permettant de piloter le système de mise en forme 3, le scanner optique de balayage 4 et le système optique de focalisation 5.

Le système de mise en forme 3 permet de moduler la phase du faisceau L.A.S.E.R. 11 issu du laser femtoseconde 1 pour répartir l'énergie du faisceau L.A.S.E.R. en une pluralité de points d'impact dans son plan focal, cette pluralité de points d'impact générés simultanément définissant un motif.

Le scanner optique de balayage 4 permet d'orienter le faisceau L.A.S.E.R. modulé en phase 31 issu du système de mise en forme 3 pour déplacer le motif de découpe le long d'un chemin de déplacement prédéfini par l'utilisateur dans le plan de focalisation 21.

Le système optique de focalisation 5 permet de déplacer le plan de focalisation 21 - correspondant au plan de découpe - du faisceau L.A.S.E.R. modulé et dévié 41.

Ainsi, le système de mise en forme 3 permet de générer simultanément plusieurs points d'impact définissant un motif, le scanner optique de balayage 4 permet de déplacer ce motif dans le plan de focalisation 21, et le système optique de focalisation 5 permet de déplacer le plan de focalisation 21 en profondeur de sorte à générer des découpes dans des plans successifs définissant un volume.

Les différents éléments constituant l'appareil de découpe vont maintenant être décrits plus en détails en référence aux figures.

## 2. *Eléments de l'appareil de découpe*

### 2.1. *Système de mise en forme*

Le système de mise en forme spatiale 3 du faisceau L.A.S.E.R. permet de faire varier la surface d'onde du faisceau L.A.S.E.R. pour obtenir des points d'impact séparés les uns des autres dans le plan focal. Plus précisément, le système de mise en forme 3 permet de moduler la phase du faisceau L.A.S.E.R. 11 issu du laser femtoseconde 1 de sorte à obtenir un unique faisceau L.A.S.E.R. modulé en phase selon une consigne de modulation calculée pour former des pics d'intensité dans le plan focal du faisceau, chaque pic d'intensité produisant un point d'impact respectif dans le plan focal correspondant au plan de découpe.

Le fait de disposer d'un unique faisceau L.A.S.E.R. modulé facilite l'intégration d'un système de balayage - tel qu'un scanner optique - pour déplacer la pluralité de faisceaux L.A.S.E.R. secondaires dans un plan de découpe. En effet, le diamètre d'entrée d'un système de balayage étant de l'ordre du diamètre du faisceau L.A.S.E.R. initiale, l'utilisation d'un unique faisceau L.A.S.E.R. modulé (dont le diamètre est sensiblement égal au diamètre du faisceau L.A.S.E.R. initial) limite les risques d'aberration qui peuvent se produire avec la technique de subdivision de faisceau telle que décrite dans US 2010/0133246.

Le système de mise en forme 3 est, selon le mode de réalisation illustré, un modulateur spatial de lumière à cristaux liquides, connu sous le sigle SLM, de l'acronyme anglais « *Spatial Light Modulator* ». Les inventeurs ont en effet découvert que l'utilisation d'un SLM était avantageuse malgré les enseignements de l'art antérieur qui dissuadent l'homme du métier d'utiliser un tel dispositif (voir notamment le paragraphe [0024] du document US 2015/0164689).

Le SLM permet de moduler la répartition finale d'énergie du faisceau L.A.S.E.R., notamment dans le plan focal 21 correspondant au plan de découpe du tissu 2. Plus précisément, le SLM est adapté pour modifier le profil spatial du front d'onde du faisceau L.A.S.E.R. primaire 11 issu du laser femtoseconde 1 pour distribuer l'énergie du faisceau L.A.S.E.R. en différents spots de focalisation dans le plan de focalisation. Ce dispositif permet de limiter les coûts associés à la modulation de la phase du front d'onde et résout ainsi les problématiques liées à l'industrialisation de la solution proposée.

La modulation en phase du front d'onde peut être vue comme un phénomène d'interférences en deux dimensions. Chaque portion du faisceau L.A.S.E.R. initial issu de la source est retardée ou avancée par rapport au front d'onde initial afin que chacune de ces portions soit redirigée de façon à réaliser une interférence constructive en N points distincts dans le plan focal d'une lentille. Cette redistribution d'énergie en une pluralité de points d'impact n'a lieu que dans un seul plan (i.e. le plan de focalisation) et pas tout au long du chemin de propagation du faisceau L.A.S.E.R. modulé. Ainsi, l'observation du faisceau L.A.S.E.R. modulé avant ou après le plan de focalisation ne permet pas d'identifier une redistribution de l'énergie en une pluralité de points d'impact distincts, du fait de ce phénomène qu'on peut assimiler à des interférences constructives (qui n'ont lieu que dans un plan et pas tout au long de la propagation comme dans le cas de la séparation d'un faisceau L.A.S.E.R. initial en une pluralité de faisceaux L.A.S.E.R. secondaires).

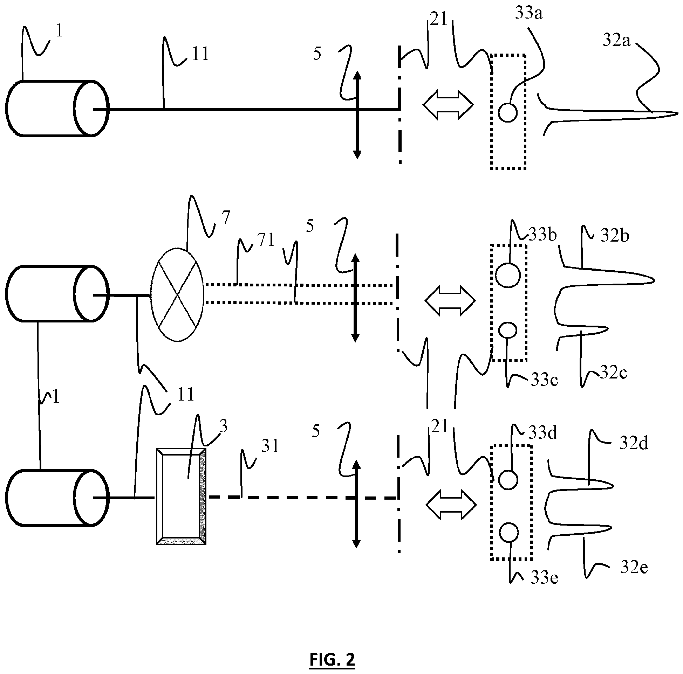

Pour mieux comprendre ce phénomène de modulation de phase du front d'onde, on a illustré schématiquement à la figure 2 des profils d'intensité 32a-32e obtenus pour trois exemples de montages optiques distincts. Comme représenté à la figure 2, un faisceau L.A.S.E.R. 11 émis par une source laser 1 produit un pic d'intensité 32a de forme gaussienne en un point d'impact 33a dans un plan de focalisation 21. L'insertion d'un séparateur de faisceau 7 entre la source 1 et le plan de focalisation 21 induit la génération d'une pluralité de faisceau L.A.S.E.R. secondaires 71, chaque faisceau L.A.S.E.R. secondaire 71 produisant un point d'impact 33b, 33c respectif dans le plan de focalisation 21 des faisceaux L.A.S.E.R. secondaires 71. Enfin, l'insertion entre la source 1 et le plan de focalisation 21 d'un SLM 3 programmé à l'aide d'un masque de phase formant consigne de modulation induit la modulation de la phase du front d'onde du faisceau L.A.S.E.R. 11 issu de la source 1. Le faisceau L.A.S.E.R. 31 dont la phase du front d'onde a été modulée permet d'induire la production de plusieurs pics d'intensité 33d, 33e séparés spatialement dans le plan focal 21 du faisceau L.A.S.E.R., chaque pic 32d, 32e correspondant à un point d'impact 33d, 33e respectif réalisant une découpe. La technique de modulation de la phase du front d'onde permet de générer plusieurs bulles de cavitation simultanées sans démultiplication du faisceau L.A.S.E.R. initial produit par le laser femtoseconde 1.

Le SLM est un dispositif constitué d'une couche de cristaux liquides à orientation contrôlée permettant de façonner d'une manière dynamique le front d'onde, et donc la phase du faisceau L.A.S.E.R. La couche de cristaux liquides d'un SLM est organisée comme une grille (ou matrice) de pixels. L'épaisseur optique de chaque pixel est contrôlée électriquement par orientation des molécules de cristal liquide appartenant à la surface correspondant au pixel. Le SLM (9) exploite le principe d'anisotropie des cristaux liquides, c'est-à-dire la modification de l'indice des cristaux liquides, en fonction de leur orientation spatiale. L'orientation des cristaux liquides peut être réalisée à l'aide d'un champ électrique. Ainsi, la modification de l'indice des cristaux liquides modifie le front d'onde du faisceau L.A.S.E.R. (4).

D'une manière connue, le SLM met en oeuvre un masque de phase, c'est-à-dire une carte déterminant comment la phase du faisceau doit être modifiée pour obtenir une répartition d'amplitude donnée dans son plan de focalisation. Le masque de phase est une image bidimensionnelle dont chaque point est associé à un pixel respectif du SLM. Ce masque de phase permet de piloter l'indice de chaque cristal liquide du SLM en convertissant la valeur associée à chaque point du masque - représentée en niveaux de gris compris entre 0 et 255 (donc du noir au blanc) - en une valeur de commande - représentée en une phase comprise entre 0 et $2\pi$. Ainsi, le masque de phase est une consigne de modulation affichée sur le SLM pour entraîner en réflexion un déphasage spatial inégal du faisceau L.A.S.E.R. (4) illuminant le SLM. Bien entendu, l'homme du métier appréciera que la plage de niveau de gris peut varier en fonction du modèle de SLM utilisé. Par exemple dans certains cas, la plage de niveau de gris peut être comprise entre 0 et 220. Le masque de phase est généralement calculé par :

- un algorithme itératif basé sur la transformée de Fourier, tel qu'un algorithme de type *« IFTA »*, acronyme de l'expression anglo-saxonne « *Iterative Fourrier Transform Algorithm »,* ou par
- divers algorithmes d'optimisation, tels que des algorithmes génétiques, ou le recuit simulé.

Ceci permet de maitriser l'homogénéité, l'intensité, la qualité et la forme des différents points d'impact générés dans le plan de découpe.

Différents masques de phase peuvent être appliqués au SLM en fonction du nombre et de la position des points d'impact souhaités dans le plan focal du faisceau L.A.S.E.R. Dans tous les cas, l'homme du métier sait calculer une valeur en chaque point du masque de phase pour distribuer l'énergie du faisceau L.A.S.E.R. en différents spots de focalisation dans le plan de focal.

Le SLM permet donc, à partir d'un faisceau L.A.S.E.R. gaussien générant un unique point d'impact, et au moyen du masque de phase, de répartir son énergie par modulation de phase de sorte à générer simultanément plusieurs points d'impact dans son plan de focalisation à partir d'un unique faisceau L.A.S.E.R. mis en forme par modulation de phase (un seul faisceau en amont et en aval du SLM).

En plus d'une diminution du temps de découpe de la cornée, la technique de modulation de la phase du faisceau L.A.S.E.R. selon l'invention permet d'autres améliorations, telles qu'une meilleure qualité de surface après découpe ou une diminution de la mortalité endothéliale. Les différents points d'impact du motif peuvent, par exemple, être régulièrement espacés sur les deux dimensions du plan focal du faisceau L.A.S.E.R., de manière à former un quadrillage de spots L.A.S.E.R.

Ainsi, le système de mise en forme 3 permet de réaliser une opération de découpe chirurgicale d'une manière rapide et efficace. Le SLM permet de façonner d'une manière dynamique le front d'onde du faisceau L.A.S.E.R. puisqu'il est paramétrable numériquement. Cette modulation permet la mise en forme du faisceau L.A.S.E.R. d'une manière dynamique et reconfigurable.

Le SLM peut être configuré pour mettre en forme le front d'onde du faisceau L.A.S.E.R. de toute autre manière. Par exemple, chaque point d'impact peut présenter une forme géométrique quelconque, autre que circulaire (par exemple en ellipse, etc.). Ceci peut présenter certains avantages en fonction de l'application considérée, comme une augmentation de la vitesse et/ou de la qualité de la découpe.

### 2.2. *Scanner optique de balayage*

Le scanner optique de balayage 4 permet de dévier le faisceau L.A.S.E.R. modulé en phase 31 de sorte à déplacer le motif 8 en une pluralité de positions 43a-43c dans le plan de focalisation 21 correspondant au plan de découpe.

Le scanner optique de balayage 4 comprend :

- un orifice d'entrée pour recevoir le faisceau L.A.S.E.R. modulé en phase 31 issu de l'unité de mise en forme 3,
- un (ou plusieurs) miroir(s) optique(s) pivotant autour d'au moins deux axes pour dévier le faisceau L.A.S.E.R. modulé en phase 31, et
- un orifice de sortie pour envoyer le faisceau L.A.S.E.R. modulé dévié 41 vers le système optique de focalisation 5.

Le scanner optique 4 utilisé est par exemple une tête de balayage IntelliScan III de la société SCANLAB AG.

Les orifices d'entrée et de sortie d'un tel scanner optique 4 présentent un diamètre de l'ordre de 10 à 20 millimètres, et les vitesses de balayage atteignables sont de l'ordre de 1m/s à 10 m/s.

Le (ou les) miroir(s) est (sont) connecté(s) à un (ou des) moteur(s) pour permettre leur pivotement. Ce(s) moteur(s) pour le pivotement du (ou des) miroir(s) est (sont) avantageusement piloté(s) par l'unité de l'unité de commande 6 qui sera décrite plus en détails dans la suite.

L'unité de commande 6 est programmée pour piloter le scanner optique de balayage 4 de sorte à déplacer le motif 8 le long d'un chemin de déplacement 42 contenu dans le plan de focalisation 21. Dans certains modes de réalisation, le chemin de déplacement 42 comprend une pluralité de segments de découpe 42a-42c. Le chemin de déplacement 42 peut avantageusement présenter une forme de créneau. Dans ce cas, si le scanner optique 4 débute un premier segment de découpe 42a par la gauche, il débutera le deuxième segment de découpe 42b par la droite, puis le troisième segment de découpe 42c par la gauche, puis le segment suivant par la droite et ainsi de suite sur tout le chemin de déplacement 42 du motif 8. Ceci permet d'accélérer la découpe du tissu en évitant la nécessité pour le scanner optique 4 de repositionner le motif 8 au début de chaque segment de découpe 42a-42c successif.

Pour accélérer encore l'opération de découpe dans le plan de focalisation 21, le chemin de déplacement 42 peut avantageusement présenter une forme de spirale. Ceci permet de maintenir constante la vitesse de balayage du scanner optique 4 dans tout le plan de découpe. En effet, dans le cas d'un chemin de déplacement 42 en forme de créneau, le scanner optique 4 doit s'arrêter à la fin de chaque segment de découpe 42a pour se déplacer sur le segment de découpe suivant 42b, ce qui consomme du temps.

Le balayage du faisceau a une influence sur le résultat de la découpe obtenu. En effet, la vitesse de balayage utilisée, ainsi que le pas du balayage, sont des paramètres influençant la qualité de la découpe.

De préférence, le pas de balayage - correspondant à la distance « dist » entre deux positions adjacentes 43a, 43b du motif 8 le long d'un segment du chemin de déplacement 42 - est choisi supérieur ou égal au diamètre d'un point d'impact 81 du motif 8. Ceci permet de limiter les risques de superposition des points d'impact lors de tirs successifs.

Egalement, lorsque le chemin de déplacement 42 présente une forme en créneau, la distance « éc » entre deux segments adjacents 42a, 42b du chemin de déplacement 42 est de préférence choisie supérieure à la dimension du motif 8 selon une perpendiculaire à sa direction de déplacement. Ceci permet aussi de limiter les risques de superposition des points d'impact 81 lors de tirs successifs.

Enfin, pour limiter la durée de l'opération de découpe dans le plan de découpe, tout en garantissant une certaine qualité de la découpe, la distance entre deux segment adjacents 42a, 42b du chemin de déplacement 42 peut être choisie égale à un maximum (et de préférence inférieure) de 3N fois le diamètre d'un point d'impact 81, où N est le nombre de points d'impact du motif 8.

Dans un mode de réalisation, l'appareil de découpe comprend en outre un prisme de Dove. Celui-ci est avantageusement positionné entre le système de mise en forme 3 et le scanner optique de balayage 4. Le prisme de Dove permet de mettre en oeuvre une rotation du motif 8 qui peut être utile dans certaines applications ou pour limiter la taille de la zone d'amorçage de chaque segment de découpe 42a-42c.

Avantageusement, l'unité de commande 6 peut être programmée pour activer le laser femtoseconde 1 lorsque la vitesse de balayage du scanner optique 4 est supérieure à une valeur seuil.

Ceci permet de synchroniser l'émission du faisceau L.A.S.E.R. 11 avec le balayage du scanner optique de balayage 4. Plus précisément, l'unité de commande 6 active le laser femtoseconde 1 lorsque la vitesse de pivotement du (ou des) miroir(s) du scanner optique 4 est constante. Ceci permet d'améliorer la qualité de découpe par la réalisation d'un surfaçage homogène du plan de découpe.

### 2.3. *Système optique de focalisation*

Le système optique de focalisation 5 permet de déplacer le plan de focalisation 21 du faisceau L.A.S.E.R. modulé et dévié 41 dans un plan de découpe du tissu 2 désiré par l'utilisateur.

Le système optique de focalisation 5 comprend :

- un orifice d'entrée pour recevoir le faisceau L.A.S.E.R. modulé en phase et dévié issu du scanner optique de balayage,
- une (ou plusieurs) lentille(s) motorisée(s) pour permettre son (leur) déplacement en translation le long du chemin optique du faisceau L.A.S.E.R. modulé en phase et dévié, et
- un orifice de sortie pour envoyer le faisceau L.A.S.E.R. focalisé vers le tissu à traiter.

La (ou les) lentilles utilisées avec le système optique de focalisation 5 peuvent être des lentilles f-theta ou des lentilles télécentriques. Les lentilles f-theta et télécentriques permettent d'obtenir un plan de focalisation sur tout le champ XY, contrairement aux lentilles standard pour lesquelles il est courbe. Cela permet de garantir une taille de faisceau focalisé constante sur tout le champ. Pour les lentilles f-theta, la position du faisceau est directement proportionnelle à l'angle appliqué par le scanner tandis que le faisceau est toujours normal à l'échantillon pour les lentilles télécentriques.

L'unité de commande 6 est programmée pour piloter le déplacement de la (ou des) lentille(s) du système optique de focalisation 5 le long d'un chemin optique du faisceau L.A.S.E.R. de sorte à déplacer le plan de focalisation 21 en au moins trois plans de découpe respectifs 22a-22e de sorte à former un empilement de plans de découpe du tissu 2. Ceci permet d'effectuer une découpe dans un volume 23, par exemple dans le cadre d'une chirurgie réfractive.

L'unité de de commande 6 est apte à piloter le déplacement du système optique de focalisation 5 pour déplacer le plan de focalisation 21 entre une première position extrême 22a et une deuxième position extrême 22e, dans cet ordre. Avantageusement, la deuxième position extrême 22e est plus proche du laser femtoseconde 1 que la première position extrême 22a.

Ainsi, les plans de découpe 22a-22e sont formés en commençant par le plan de découpe le plus profond 22a dans le tissu et en empilant les plans de découpe successifs jusqu'au plan de découpe le plus superficiel 22e dans le tissu 2. On évite ainsi les problèmes associés à la pénétration du faisceau L.A.S.E.R. dans le tissu 2. En effet, les bulles de cavitation forment une barrière de bulles opaque (connue sous le nom de « OBL », sigle de l'expression anglaise « Opaque Bubble Layer») empêchant la propagation de l'énergie issue du faisceau L.A.S.E.R. sous celles-ci. Il est donc préférable de commencer par générer les bulles de cavitation les plus profondes en priorité afin d'améliorer l'efficacité de l'appareil de découpe.

De préférence, la longueur du chemin optique entre le système de mise en forme 3 et le système optique de focalisation 5 est inférieure à 2 mètres, et encore plus préférentiellement inférieure à 1 mètre. Ceci permet de limiter les pertes de puissance dues à de l'énergie dispersée sur le chemin optique. En effet plus la distance entre le système de mise en forme 3 et le système optique de focalisation 5 est grande, plus la perte de puissance sur le trajet est importante.

Avantageusement, l'unité de commande 6 peut être programmée pour faire varier la forme du motif 8 entre deux plans de découpe 22a-22b (ou 22b-22c, ou 22c-22d, ou 22d-22e) successifs. En effet, lors de la découpe dans un volume 23, il peut être préférable d'augmenter la précision de la découpe dans les plans de découpe périphériques 22a, 22e et d'augmenter la vitesse de découpe dans les plans de découpe intermédiaires 22b, 22c, 22d situés entre les plans de découpe périphériques 22a, 22e. Par exemple dans le cas de la découpe d'un volume 23 composé d'un empilement de cinq plans de découpe 22a-22e, l'unité de commande 6 peut piloter le système de mise en forme 3 en lui transmettant :

- un premier masque de phase correspondant à un premier motif permettant d'augmenter la précision de la découpe lorsque le plan de focalisation correspond aux premier et cinquième plans de découpe 22a et 22e,
- un deuxième masque de phase lorsque le plan de focalisation correspond aux deuxième, troisième et quatrième plans de découpe 22b-22d.

De même, l'unité de commande 6 peut être programmée pour faire varier le pas « *dist* » du scanner optique de balayage 4 et/ou la forme de la zone découpée (en modifiant le chemin de déplacement du motif) entre deux plans de découpe respectifs. Ceci permet également soit d'augmenter la précision de la découpe, soit la vitesse de découpe dans d'un plan de découpe à un autre.

Enfin, l'unité de commande 6 peut être programmée pour piloter le scanner optique de balayage 4 de sorte à faire varier l'aire découpée dans le plan de focalisation 21 entre deux plans de découpe successifs 22d, 22e. Ceci permet de faire varier la forme du volume 23 finalement découpé en fonction de l'application visée.

De préférence, la distance entre deux plans de découpe successifs est comprise entre 2 μm et 500 μm, et notamment :

- entre 2 et 20μm pour traiter un volume nécessitant une grande précision, par exemple en chirurgie réfractive, avec de préférence un espacement compris entre 5 et 10μm, ou
- entre 20 et 500μm pour traiter un volume ne nécessitant pas une grande précision, comme par exemple pour détruire la partie centrale d'un noyau cristallinien, avec de préférence un espacement compris entre 50 et 200μm.

Bien entendu, cette distance peut varier dans un volume 23 composé d'un empilement de plans de découpe 22a-22e.

*2.4. Filtre*

L'appareil de découpe peut également comprendre un filtre disposé en aval du système de mise en forme 3.

Le filtre permet d'une part de bloquer l'énergie « parasite » générée au centre du système de mise en forme 3 (phénomène connu sous la dénomination « ordre zéro »). En effet, lors de la modulation en phase du faisceau L.A.S.E.R. avec le système de mise en forme, une partie du faisceau L.A.S.E.R. issu de la source laser 1 n'est pas modulé (du fait de l'espace existant entre les pixels des cristaux liquides du SLM). Cette partie du faisceau L.A.S.E.R. non modulé peut induire la génération d'un pic d'énergie se formant au centre du SLM.

Le filtre permet d'autre part de limiter les risques de lésions L.A.S.E.R. inopinée pour le patient en cas de défaillance du système de mise en forme 3. En effet, si le système de mise en forme 3 est défectueux, le faisceau L.A.S.E.R. n'est pas modulé, ce qui induit la formation d'un pic de très forte énergie au centre du système de mise en forme 3. En bloquant ce pic de très forte énergie, le filtre permet d'éviter la génération intempestive de bulles de cavitation.

Le filtre peut être placé entre deux lentilles convergentes disposées en aval du système de mise en forme 3. En effet, l'ordre 0 ne peut être éliminé que dans un plan de Fourier (c'est à dire au foyer d'une lentille), là où la mise en forme du faisceau se produit.

Le filtre consiste par exemple en une plaque transparente au rayonnement L.A.S.E.R. sur toute sa surface à l'exception d'une région centrale de la plaque qui est opaque au rayonnement L.A.S.E.R. Pour rendre la région centrale de la plaque opaque au rayonnement L.A.S.E.R., le filtre peut comprendre une pastille opaque disposée au centre de la surface, la pastille ayant un diamètre supérieur ou égale au diamètre d'un faisceau L.A.S.E.R.

Ce filtre est alors positionné de sorte qu'une droite normale au système de mise en forme 3, et passant par le centre dudit système de mise en forme 3 passe également par la région centrale opaque au rayonnement L.A.S.E.R.

*2.5. Unité de commande*

Comme indiqué précédemment, l'unité de commande 6 permet de contrôler les différents éléments constituant l'appareil de découpe, à savoir le laser femtoseconde 1, le système de mise en forme 3, le scanner optique de balayage 4 et le système optique de focalisation 5.

L'unité de commande 6 est connectée à ces différents éléments par l'intermédiaire d'un (ou plusieurs) bus de communication permettant :

- la transmission de signaux de commande tels que
  - le masque de phase au système de mise en forme,
  - le signal d'activation au laser femtoseconde,
  - la vitesse de balayage au scanner optique de balayage,
  - la position du scanner optique de balayage le long du chemin de déplacement,
  - la profondeur de découpe au système optique de focalisation.
- la réception de données de mesure issues des différents éléments du système tels que
  - la vitesse de balayage atteinte par le scanner optique, ou
  - la position du système optique de focalisation, etc.

L'unité de commande 6 peut être composée d'une (ou plusieurs) station(s) de travail, et/ou d'un (ou plusieurs) ordinateur(s) ou peut être de tout autre type connu de l'homme du métier. L'unité de commande 6 peut par exemple comprendre un téléphone portable, une tablette électronique (tel qu'un IPAD®), un assistant personnel (ou « *PDA* », sigle de l'expression anglo-saxonne « *Personal Digital Assistant »),* etc. Dans tous les cas, l'unité de commande 6 comprend un processeur programmé pour permettre le pilotage du laser femtoseconde 1, du système de mise en forme 3, du scanner optique de balayage 4, du système optique de focalisation 5, etc.

*2.6. Motif*

La modulation reconfigurable du front d'onde du faisceau L.A.S.E.R. permet de générer de multiples points d'impact 81 simultanés ayant chacun une taille et une position contrôlée dans le plan de focalisation 21.

Ces différents points d'impact 81 forment un motif 8 dans le plan focal 21 du faisceau L.A.S.E.R. modulé.

Le nombre de points d'impact 81 du motif 8 diminue d'autant de fois le temps nécessaire à l'opération de découpe chirurgicale.

Toutefois, la dimension du motif 8, le nombre de points d'impact 81 qu'il comprend et leurs positions respectives par rapport à la direction de déplacement sont des caractéristiques techniques choisies judicieusement pour répondre à des contraintes techniques associées à la découpe de tissu, comme il ressortira dans la suite.

#### *2.6. 1. Contraintes et solutions choisies*

#### *2.6.1.1. Nombre maximal de points d'impact par motif*

Afin d'accélérer la découpe du tissu 2, il est préférable de disposer d'un motif 8 incluant un nombre maximum de points d'impact 81. Dans les systèmes laser ophtalmiques actuels, l'énergie par impulsion et par spot nécessaires à la découpe cornéenne est de l'ordre de 1μJ. Ainsi, avec un laser femtoseconde - tel qu'une source laser Satsuma (Commercialisé par Amplitude Système) - fournissant une puissance de 20W à une cadence de 500kHz, soit au maximum une énergie de 40μJ/impulsion, il est théoriquement possible de créer un motif 8 composé de 40 points d'impact 81 identiques.

Cependant, dans tout système laser, des pertes s'opèrent le long du trajet optique. Ainsi, dans un prototype testé par la Demanderesse, la puissance arrivant sur la cornée n'était plus que de 12W pour une mise en forme six points d'impact 81 de taille globale (30μm*22μm). Le diamètre de faisceau focalisé était de 8μm de diamètre, contre environs 4μm au maximum pour les lasers ophtalmiques actuels. Dans le cadre d'un prototype testé par la Demanderesse, une énergie 4 fois plus importante par spot a été nécessaire par rapport aux lasers ophtalmiques actuels, soit 4μJ. Ainsi pour ce prototype, il a été choisi d'utiliser un motif composé de six points d'impact 81 (au maximum). Bien entendu, si la puissance du laser femtoseconde 1 est supérieure, le motif 8 peut comprendre un nombre de points d'impact 81 supérieur à six.

#### *2.6.1.2. Distribution des points d'impact dans le motif*

Les six points d'impact 81 du motif 8 peuvent être répartis selon différentes configurations.

Par exemple les six points d'impact 81 peuvent être répartis le long d'une ligne unique. La longueur totale du motif 8 est alors égale à la somme entre le diamètre d'un point d'impact 81 et la distance centre à centre entre les points d'impact 81 extrêmes du motif 8. La largeur du motif 8 est quant à elle égale au diamètre d'un point d'impact 81.

Comme indiqué précédemment, la mise en forme du faisceau L.A.S.E.R. entraine une perte de puissance, due à de l'énergie dispersée sur le chemin optique. La taille globale de la mise en forme (et donc la taille du motif 8) fait partie des facteurs influençant cette perte d'énergie.

Plus la taille (en longueur ou en largeur) du motif 8 est grande, et plus la perte de puissance est grande. Une répartition de six points d'impact 81 sur une ligne unique induit donc une perte de puissance importante.

A titre indicatif :

- un motif 8 de taille 30μm*22μm comprenant six points d'impact 81 entraine une perte de puissance de 10% environ, tandis que
- un motif 8 de taille 84μm*20μm comprenant cinq points d'impact 81 entraine une perte de puissance de 25% environ.

Ainsi, pour un nombre de points d'impact 81 donné, les motifs « *compacts* » (rapport des tailles en longueur et en largeur proche de 1) entrainent une perte d'énergie plus faible.

C'est pourquoi les points d'impact 81 du motif 8 sont de préférence compris dans une surface dont le rapport entre la longueur et la largeur est compris entre 1 et 4, préférentiellement entre 1 et 2, et encore plus préférentiellement entre 1 et 1.5.

Par exemple, les six points d'impact 81a-81f du motif peuvent être répartis sur des première et deuxième lignes 82, 83 parallèles :

- la première ligne 82 passant par trois points d'impact 81a-81c formant un premier triplet,
- la deuxième ligne 83 passant par trois autres points d'impact 81d-81f formant un deuxième triplet distinct du premier triplet.

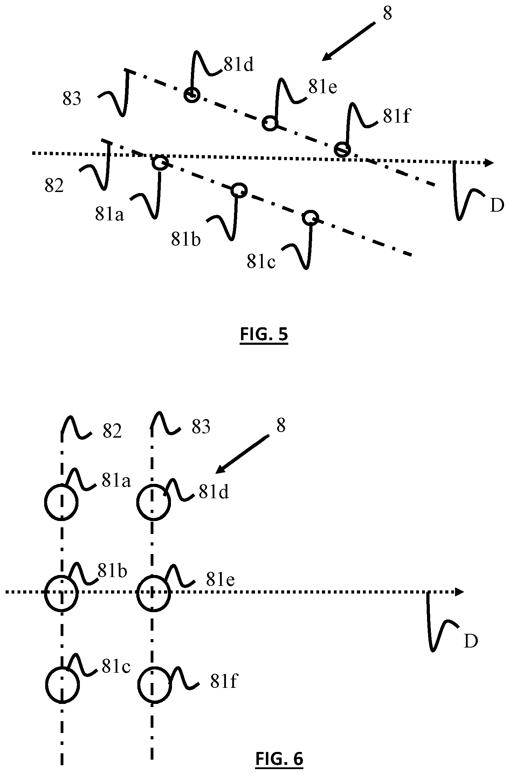

Un motif correspondant à cette distribution est illustré à la figure 5. Avantageusement, les points d'impacts 81a-81f du motif peuvent être décalés d'une ligne à l'autre selon la direction de déplacement D. Plus précisément, les points d'impact 81a-81c du premier triplet peuvent être décalés d'une distance non nulle (selon la direction de déplacement D) par rapport aux points d'impact 81d-81f du deuxième triplet. Ceci permet d'éviter la superposition de bulles de cavitation dans le plan de découpe lors du déplacement du motif 8 par le scanner optique de balayage 4.

*2.6.1.3. Distance minimale entre points d'impact du motif*

Outre la distribution des points d'impacts 81 du motif 8, un autre paramètre du motif concerne la distance entre les points d'impact adjacents.

Cette distance est définie par des contraintes liées au système de mise en forme.

Lors de l'opération de mise en forme du faisceau L.A.S.E.R. issu du laser femtoseconde, les points d'impact « *trop proches* » interfèrent entre eux du fait de la cohérence spatiale de la source. Ces interférences dégradent la forme des points d'impact et rendent impossible le contrôle du niveau d'intensité laser sur chaque point d'impact. Il est donc préférable que la distance entre les points d'impact adjacents du motif soit suffisante pour limiter ce phénomène d'interférence entre points d'impact trop proches.

Cette *« distance suffisante »* dépend de la focalisation du faisceau. Plus le faisceau sera focalisé, plus cette distance sera faible. A l'inverse, moins le faisceau sera focalisé, plus cette distance sera importante.

En prenant en compte les contraintes de distance de travail liées aux applications de chirurgie du segment antérieur de l'oeil, de reproductibilité de la mise en forme ainsi que les aberrations du système optique dégradant la cohérence spatiale du faisceau, la limite de séparation de deux spots se situe à 10μm environ.

C'est pourquoi la *« distance suffisante »* de centre à centre entre deux points d'impact adjacents est supérieure à 5μm, préférentiellement supérieure à 10μm et encore plus préférentiellement comprise entre 10μm et 20μm, notamment entre 10μm et 15μm.

*2.6.1.4. Orientation du motif par rapport à la direction de déplacement*

La forme de base illustrée à la figure 5 peut être orientée de différentes façons dans le maillage.

L'orientation la plus évidente de cette forme de base pour l'homme du métier est illustrée à la figure 6. Cette orientation consiste à déplacer le motif selon une direction de déplacement D perpendiculaire aux deux lignes 82, 83 définies par les premier et deuxième triplets de points d'impact 81a-81c et 81d-81f.

Cependant, plusieurs limitations liées au système de mise en forme et à la direction de déplacement du motif empêchent l'utilisation d'une telle orientation.

Comme décrit précédemment, la distance entre deux points d'impact adjacents 81a, 81b du motif est préférentiellement supérieure à 10 μm. En déplaçant le motif selon une direction de déplacement perpendiculaire aux deux lignes 82, 83 définies par les premier et deuxième triplets de points d'impact 81a-81c, 81d-81f, la distance entre les bulles de cavitations générées sur des segments adjacents 42a, 42b parallèles à la direction de déplacement du motif sera de l'ordre de 15 μm.

Or, une distance « *classique »* entre bulles de cavitation adjacentes pour la découpe d'une cornée est de l'ordre de 2μm à 7 μm, notamment égale à 5μm.

Il est donc nécessaire « *d'incliner»* le motif 8 de sorte que les bulles de cavitations voisines générées sur des segments 42a, 42b adjacents parallèles à la direction de déplacement D du motif 8 soient espacées d'une distance sensiblement égale à 5μm à la direction de déplacement.

On notera que sur un même segment 42a, la distance de 5μm entre deux bulles de cavitations adjacentes peut être obtenue en réglant le pas de déplacement du scanner optique de balayage 4.

*2.6.2. Exemples de motifs retenus*

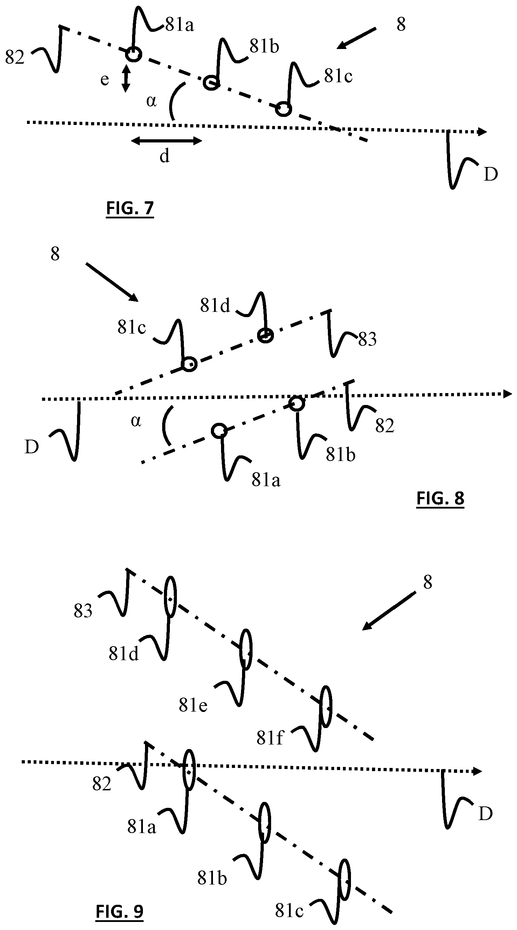

En référence aux figures 7 à 9, on a illustré différents exemples de motif utilisable avec l'appareil de découpe selon l'invention.

Dans le mode de réalisation illustré à la figure 7, le motif comprend trois points d'impact 81a-81c s'étendant le long d'une ligne 82 du motif 8. Les points d'impact sont espacés d'une distance « *d »* selon la direction de déplacement D. La ligne du motif est inclinée d'un angle *« $\alpha$ »* par rapport à la direction de déplacement D du scanner optique de balayage 4 de sorte que les bulles de cavitation selon une droite perpendiculaire à la direction de déplacement D sont espacées d'une distance « e » dans le plan de découpe. On a alors la relation suivante entre les différentes distances « *d »* et « e », et l'angle d'inclinaison « $\alpha$ *» :*

$$\propto \; = \tan^{-1}\left(\frac{e}{d}\right)$$

De préférence, l'angle d'inclinaison « $\alpha$ » du motif est compris entre 10° et 80°.

Dans le mode de réalisation illustré à la figure 8, le motif comprend quatre points d'impact 81a-81d s'étendant le long de deux lignes parallèle 82, 83 du motif 8 :

- Une première paire de points d'impact 81a, 81b s'étend le long d'une première ligne 82 du motif,
- Une deuxième paire de points d'impact 81c, 81d s'étend le long d'une deuxième ligne 83 du motif.

Ce motif présente une forme de carré incliné d'un angle d'inclinaison « $\alpha$ » par rapport à la direction de déplacement du scanner optique de balayage. On a la relation suivante :

$$\propto \; = \tan^{-1}\left(\frac{e}{d}\right)$$

Avec :

- « $\alpha$ » l'angle d'inclinaison de chaque ligne du motif par rapport à la direction de déplacement,
- « *d* » correspondant à la distance entre deux points d'impact adjacents, et
- « *e* » correspondant à la distance entre deux points d'impact adjacents selon une direction perpendiculaire à la direction de déplacement du motif.

Dans le mode de réalisation illustré à la figure 9, le motif comprend six points d'impact 81a-81f s'étendant le long de deux lignes parallèle du motif 8 :

- Un premier triplet de points d'impact s'étend le long d'une première ligne du motif,
- Un deuxième triplet de points d'impact s'étend le long d'une deuxième ligne du motif.

Ce motif présente une forme de rectangle incliné d'un angle d'inclinaison « $\alpha$ » par rapport à la direction de déplacement du scanner optique de balayage. On a la relation suivante :

$$\propto \; = \tan^{-1}\left(\frac{e}{d}\right)$$

Avec :

- « $\alpha$ » l'angle d'inclinaison de chaque ligne du motif par rapport à la direction de déplacement,
- « d » correspondant à la distance entre deux points d'impact adjacents, et « e » correspondant à la distance entre deux points d'impact adjacents selon une direction perpendiculaire à la direction de déplacement du motif.

*2.6.3. Théorie relative à la détermination de motifs*

Dans la suite, on va décrire une démarche mises en oeuvre par la Demanderesse pour déterminer des formes possibles des motifs de points d'impact permettant d'obtenir au final un arrangement de bulles de cavitations composé d'une matrice régulière répétitive :

- soit en carré,
- soit en triangle équilatéral,

tout en respectant l'espacement minimum entre points d'impact adjacents pour limiter le phénomène d'interférence décrit précédemment.

Il existe une variété de motifs possibles pour obtenir par projection lors de leur déplacement une matrice homogène et répétitive de bulles de cavitation distantes de 5$\mu$m les unes des autres, sur toute la surface traitée. Mais il existe aussi une matrice « idéale » dont les points d'impact sont suffisamment éloignés pour éviter les interférences, et suffisamment proches pour que la surface totale du motif soit faible et s'inscrive dans un champ restreint, ce qui est préférable en raison de la taille limitée des optiques et des miroirs qui se trouvent sur le trajet du faisceau L.A.S.E.R.

Nous avons procédé simplement par l'observation d'un arrangement de spots, soit avec une matrice carrée, soit avec une matrice en triangle équilatéral, et nous avons déterminé les motifs possibles pour obtenir cet arrangement, une fois le motif mis en mouvement par le scanner optique de balayage.

*2.6.3.1. Recherche d'un motif pour obtenir un arrangement de bulles de cavitation en matrice triangle équilatérale*

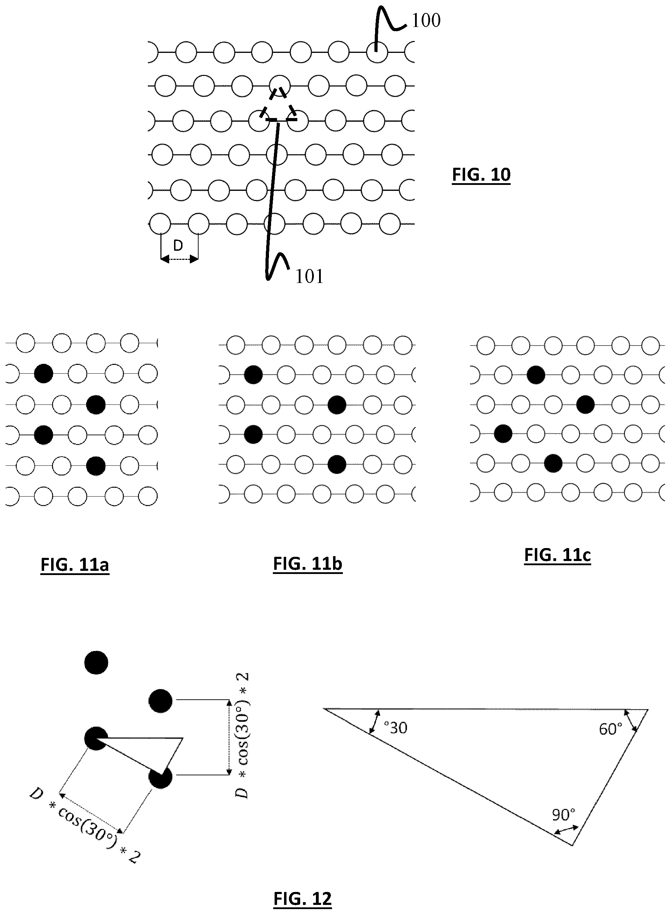

Dans la figure 10 illustrant un plan découpé incluant une pluralité de bulles de cavitation 100, on peut observer un arrangement de bulles en triangle équilatéral formant matrice 101.

L'observation, nous conduit à identifier plusieurs motifs possibles, qui s'inscrivent dans cette matrice, comme illustré sur les figures 11a à 11c.

En pratique aucune des trois matrices présentées ci-dessus n'est utilisable. En effet, si la distance séparant 2 bulles de la surface découpée est D, ou 5μm, il faut aussi pour éviter les interférences que la distance minimale entre 2 points d'impact du motif soit égale à 10μm, soit au minimum 2D.

Or dans les trois exemples de motifs illustrés aux figures 11a à 11c, il y a toujours au moins deux points d'impact du motif trop proches les uns des autres (distance = D * (Cos(30°) * 2) = 1,73 * D. Soit pour D = 5 μm une distance de 8,65 μm (cf. figure 12).

Ces observations nous conduisent donc à définir un motif dont tous les points d'impact sont au minimum distants les uns des autres de 2 * D et qui permette d'obtenir l'arrangement en motif triangle équilatéral.

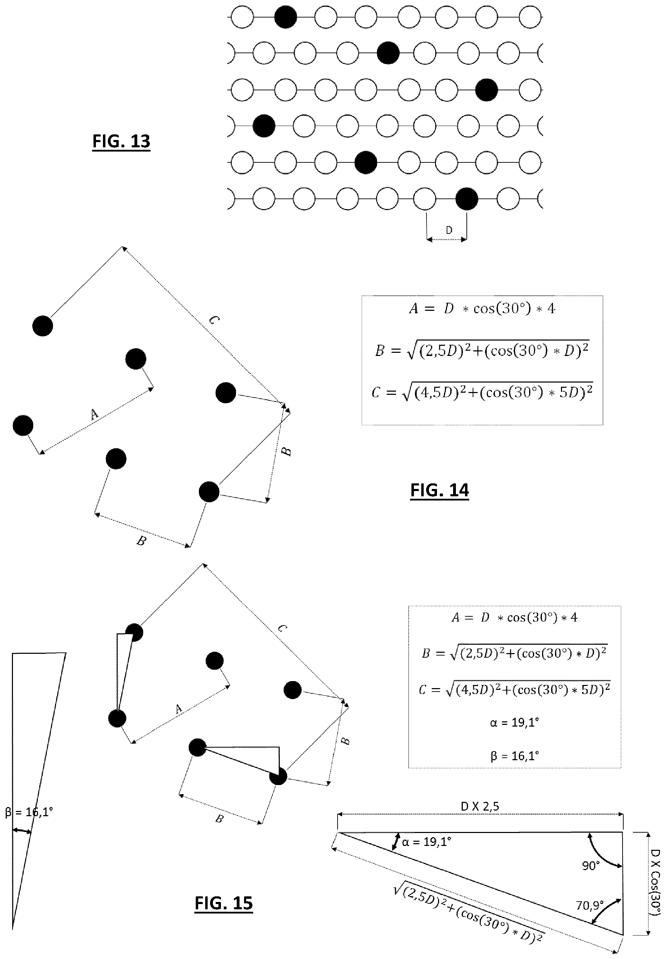

Un premier exemple de motif est illustré aux figures 13 à 15, dans lequel tous les points sont distants les uns des autres au minimum de 2 * D, soit pour les distances A et B si D = 5μm :

- $$A = D * \cos(30°) * 4 = 17\ \mu m$$
- $$B = \sqrt{(2{,}5D)^2 + (\cos(30°) * D)^2} = 13{,}22\ \mu m$$

D'autre part, la distance entre les deux points les plus espacés de la matrice est de

$$C = \sqrt{(4{,}5D)^2 + (\cos(30°) * 5D)^2} = 31{,}22\ \mu m$$

Enfin, sur cette matrice, une angulation précise (cf. figure 15) permet de reproduire le motif régulier en triangle équilatéral, et les angles par rapport à l'horizontale et la verticale sont :

$$A = D * \cos(30°) * 4$$

$$B = \sqrt{(2{,}5D)^2 + (\cos(30°) * D)^2}$$

$$C = \sqrt{(4{,}5D)^2 + (\cos(30°) * 5D)^2}$$

$$\alpha = 19{,}1°$$

$$\beta = 16{,}1°$$

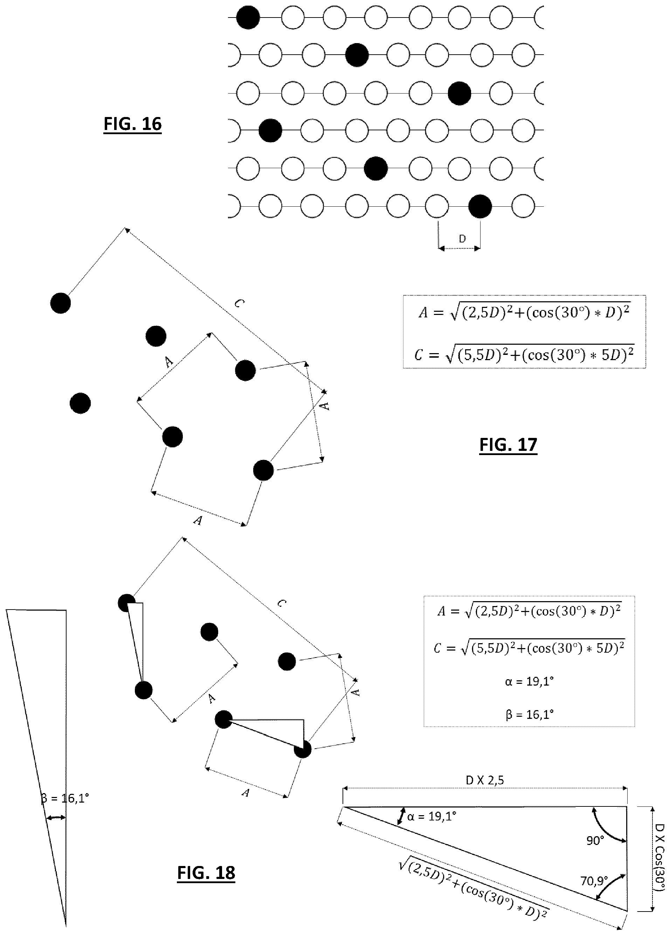

Un deuxième exemple de motif est illustré aux figures 16 à 18, dans lequel tous les points sont distants les uns des autres au minimum de 2 * D, soit pour les distances A si D = 5μm :

- $$A = \sqrt{(2{,}5D)^2 + (\cos(30°) * D)^2} = 13{,}22\ \mu m.$$

D'autre part, la distance entre les deux points les plus espacés de la matrice est de

$$C = \sqrt{(5{,}5D)^2 + (cos(30°) * 5D)^2} = 35 \mu m$$

Enfin, sur ce motif, une angulation précise permet de reproduire la matrice en triangle équilatéral, et les angles par rapport à l'horizontale et la verticale sont :

$$A = \sqrt{(2{,}5D)^2 + (\cos(30°) * D)^2}$$

$$C = \sqrt{(5{,}5D)^2 + (\cos(30°) * 5D)^2}$$

$$\alpha = 19{,}1°$$

$$\beta = 16{,}1°$$

De ce qui précède, nous venons de démontrer que deux motifs différents pouvaient être utilisés pour obtenir après mise en mouvement, un arrangement de bulles régulières disposées selon une matrice en triangle équilatéral.

Le choix entre le premier ou le deuxième motif irait plutôt en faveur du premier, car l'espacement maximum entre les 2 points les plus distants est de 31,22 µm au lieu de 35µm, donc une forme plus compacte.

Un autre intérêt de ces motifs, est que le nombre de points peut être augmenté à plus de 6 (2 × 3 points) par ajout de nouvelles rangées de points, en respectant les mêmes distances et angulations et en passant à des motifs de 9 (3 X 3) ou 12 points (3 X 4) ou plus.

*2.6.3.2. Recherche d'un motif pour obtenir un arrangement de bulles de cavitation en matrice carrée*

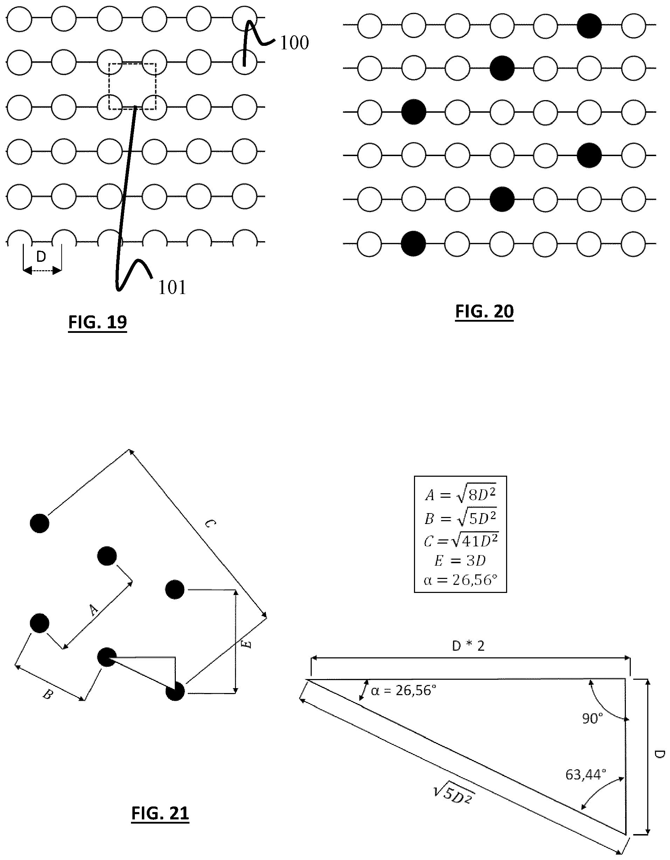

Dans la figure 19 illustrant un plan découpé incluant une pluralité de bulles de cavitation 100, on peut observer un arrangement de bulles en carré formant matrice 101.

L'observation, nous conduit à identifier un motif possible, qui s'inscrit dans cette matrice, et qui respecte l'espacement minimum entre 2 points égal à 2 fois la distance D :

- $$A = \sqrt{8D^2} = 14{,}14\ \mu m$$
- $$B = \sqrt{5D^2} = 11{,}18\ \mu m$$
- $$E = 3D = 15\ \mu m.$$

D'autre part, la distance entre les deux points les plus espacés du motif est de :

$$C = \sqrt{41D^2} = 32\ \mu m$$

Enfin, sur cette matrice, une angulation précise permet de reproduire le motif régulier en carré, et l'angle par rapport à l'horizontale est α=26.56°.

*2.6.3.3. Cas particulier de motifs entrelacés*

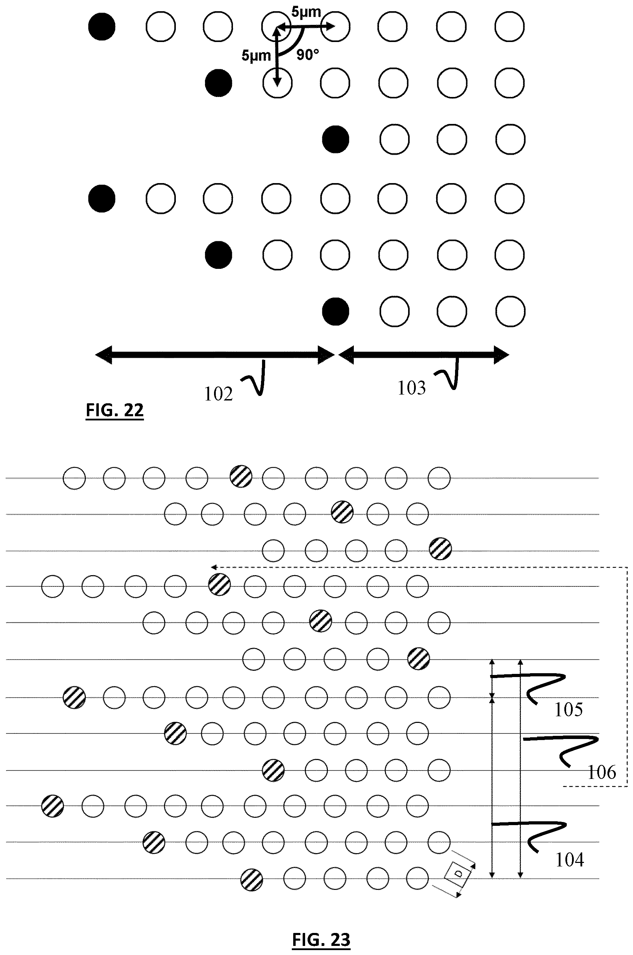

Nous avons décrit le principe de l'utilisation de motifs de spots lasers pour obtenir un arrangement homogène de bulles de cavitation dans le tissu traité. Ces motifs présentent un arrangement particulier des spots lasers, dont la disposition les uns par rapport aux autres, et les distances qui les séparent, permettent de respecter les contraintes exposées plus haut, et notamment la distance minimale entre chaque spot pour éviter les interférences, et la distance maximale entre chaque point d'impact pour obtenir une qualité de découpe du tissu satisfaisante. Les motifs présentés jusqu'à présent, ont tous la particularité de permettre lorsqu'un mouvement leur est appliqué par le balayage imprimé par le scanner, de recouvrir uniformément et de manière régulière une surface de bulles de cavitation équidistantes, sans laisser de zones non traitées. A la fin d'un segment présentant un arrangement régulier de points d'impacts, comme indiqué sur la figure 23, le scanner commande le déplacement de la matrice d'un pas 106 égal à la distance entre les rangées d'impacts les plus éloignées 104, augmentée de la distance entre deux lignes contigües 105.

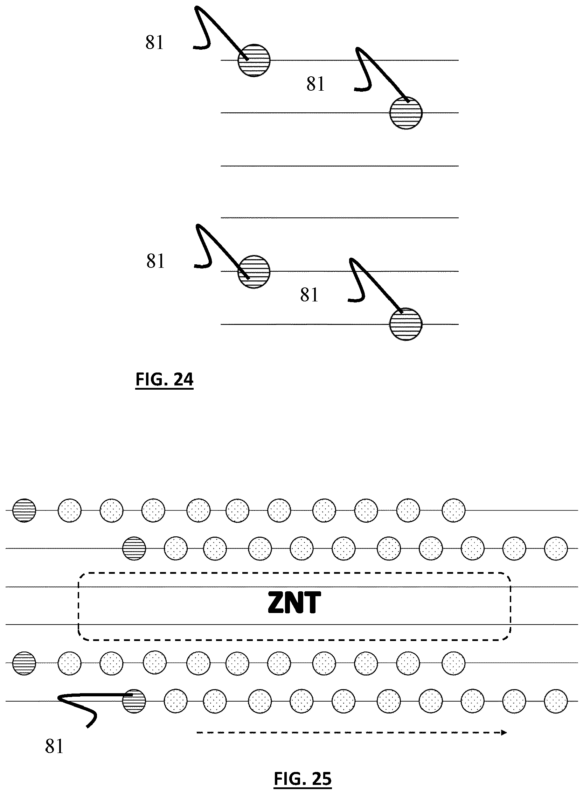

Une variante de motif présentée figure 24, permet d'imaginer de laisser une zone non traitée ZNT comme présenté figure 25, cette zone ZNT pouvant être traitée par le balayage suivant avec un arrangement de points d'impacts entrelacés. Pour cela le pas imprimé par le scanner entre deux segments successifs n'est pas constant et sera une fois sur deux égal à deux fois la distance entre deux rangées d'impacts contigües 107, et une fois sur deux égal à la distance entre les rangées d'impacts les plus éloignées 108, augmentée de la distance entre deux lignes contigües 105.

*2.6.3.4. Cas particulier d'un motif à point d'impact central*

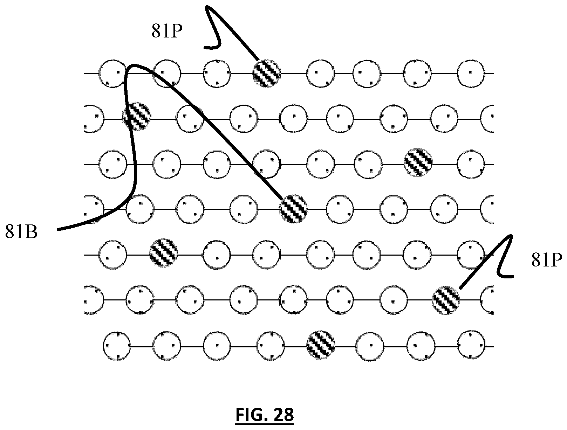

En référence à la figure 28, on a illustré un autre exemple de motif pouvant être utilisé pour découper un tissu. Ce motif comprend une pluralité (i.e. au moins trois) de points d'impact périphériques 81P, et un point d'impact central 81B positionné au barycentre du motif, Notamment dans l'exemple illustré à la figure 28, au niveau de l'intersection entre des axes diagonaux passant par des points d'impact périphérique opposés.

La présence de ce point d'impact central permet de tirer parti des phénomènes de génération d'une énergie au centre du motif (phénomène connu sous la dénomination « ordre zéro »). En effet, lors de la modulation en phase du faisceau L.A.S.E.R. 11 avec le système de mise en forme 3, une partie du faisceau L.A.S.E.R. issu du laser femtoseconde n'est pas modulé (du fait de l'espace existant entre les pixels des cristaux liquides du SLM). Cette partie du faisceau L.A.S.E.R. non modulé peut induire la génération d'un pic d'énergie se formant au centre du SLM.

Lorsque le motif ne comprend pas de point d'impact au niveau de ce barycentre, il est nécessaire de limiter ce pic d'énergie d'ordre zéro pour éviter la génération intempestive de bulles de cavitation lors du déplacement du motif dans le plan de découpe.

*2.6.3.5. Remarques*

Nous avons décrit comment disposer les points d'impact d'un faisceau laser multipoint, pour que les bulles générées présentent un arrangement homogène et régulier sur la surface de découpe du tissu. Parmi une infinité d'arrangements non réguliers, qui peuvent également être utilisés, nous avons démontré que pour obtenir un arrangement régulier en triangle équilatéral, il existait deux types de motifs préférés et que pour obtenir un arrangement régulier en carré, il existait un motif préféré. Pour toutes les matrices préférées, les espacements et angles entre chaque point de la matrice ont été calculés.

Bien évidemment, l'invention porte également sur tout type de motif dont les points d'impact sont suffisamment espacés les uns des autres pour éviter les interférences et dont le mouvement permet d'obtenir par projection une couverture relativement homogène de la surface à découper, même sans répétition régulière d'une matrice géométrique, même si les matrices présentées donnent de meilleurs résultats.

L'inconvénient de ce type de formes de motif est l'introduction d'une *« zone d'amorçage »* 102 en périphérie d'une zone régulière 103. Dans cette zone d'amorçage 102, la découpe est incomplète, comme illustré sur la figure 22. Bien que la taille de cette zone d'amorçage 102 soit très faible par rapport à la taille globale de la découpe (moins de 0.5% d'un diamètre de capot cornéen de 8mm pour les exemples présentés), cette zone d'amorçage 102 devra de préférence être la plus courte possible.

*2.6.4. Dispositif de découpe relatif au motif et procédé associé*

En résumer des paragraphes précédents concernant les différentes caractéristiques relatives au motif, les inventeurs ont proposé un appareil de découpe d'un tissu humain ou animal, tel qu'une cornée, ou un cristallin, l'appareil incluant un laser femtoseconde apte à émettre un faisceau L.A.S.E.R. sous forme d'impulsions, et un dispositif de traitement disposé en aval du laser femtoseconde pour traiter le faisceau L.A.S.E.R. généré par le laser femtoseconde, le dispositif de traitement comprenant :

- un système de mise en forme 3 positionné sur la trajectoire dudit faisceau, pour moduler la phase du front d'onde du faisceau L.A.S.E.R. de sorte à obtenir un faisceau L.A.S.E.R. modulé en phase selon une consigne de modulation calculée pour répartir l'énergie du faisceau L.A.S.E.R. en au moins deux points d'impact 81 formant un motif 8 dans son plan focal 21 correspondant à un plan de découpe, chaque point d'impact réalisant une découpe,
- un scanner optique de balayage 4 disposé en aval du système de mise en forme pour déplacer le motif dans le plan de découpe en une pluralité de positions 43 selon une direction de déplacement D,
- une unité de commande incluant un processeur programmé pour permettre le pilotage du laser femtoseconde, du système de mise en forme et du scanner optique de balayage afin d'incliner le motif par rapport à la direction de déplacement de sorte qu'au moins deux points d'impact du motif sont espacés :

- d'une distance non nulle selon un premier axe parallèle à la direction de déplacement d'une part, et
- d'une distance non nulle selon un deuxième axe perpendiculaire à la direction de déplacement d'autre part.

Avantageusement, le motif peut comprendre au moins deux (notamment trois) points d'impact adjacents s'étendant le long d'une ligne du motif, l'angle entre ladite ligne du motif et la direction de déplacement étant compris entre 10 et 80°, préférentiellement compris entre 15° et 40°, et encore plus préférentiellement entre 19° et 30°. Par ailleurs, le motif peut comprendre :

- un premier ensemble d'au moins deux (notamment trois) points d'impact disposés le long d'une première ligne du motif, et
- un deuxième ensemble d'au moins deux (notamment trois) autres points d'impact disposés le long d'une deuxième ligne du motif parallèle à la première ligne.

Le motif peut comprendre en outre au moins un autre ensemble de points d'impact disposés le long d'au moins une autre ligne du motif, parallèle aux première et deuxième lignes. Les points d'impact du deuxième ensemble peuvent être décalés d'une distance non nulle par rapport aux points d'impact du premier ensemble. En variante, chaque point d'impact du deuxième ensemble peut être aligné avec un point d'impact respectif du premier ensemble selon une droite perpendiculaire à la direction de déplacement. Avantageusement, la distance entre deux points d'impact adjacents du motif peut être supérieure à 5μm préférentiellement supérieure à 10μm et encore plus préférentiellement comprise entre 10 et 15μm. Le motif peut également être inscrit dans une surface dont le rapport entre la longueur et la largeur est compris entre 1 et 4, préférentiellement entre 1 et 2, et encore plus préférentiellement entre 1 et 1.5. Enfin, le motif peut comprendre un point d'impact central positionné au barycentre du motif.

Les inventeurs ont également proposé un procédé de commande d'un appareil de découpe incluant un laser femtoseconde apte à émettre un faisceau L.A.S.E.R. sous forme d'impulsions, et un dispositif de traitement disposé en aval du laser femtoseconde pour traiter le faisceau L.A.S.E.R., le dispositif de traitement comportant un système de mise en forme et un scanner optique de balayage, le procédé comprenant les étapes consistant à :

- moduler, en utilisant le système de mise en forme, la phase du front d'onde du faisceau L.A.S.E.R. de sorte à obtenir un faisceau L.A.S.E.R. modulé en phase selon une consigne de modulation calculée pour répartir l'énergie du faisceau L.A.S.E.R. en au moins deux points d'impact 81 formant un motif dans son plan focal correspondant à un plan de découpe, chaque point d'impact réalisant une découpe,
- déplacer, en utilisant le scanner optique de balayage, le motif dans le plan de découpe en une pluralité de positions selon une direction de déplacement D,
- incliner le motif par rapport à la direction de déplacement de sorte qu'au moins deux points d'impact du motif soient espacés :
  - d'une distance non nulle selon un premier axe parallèle à la direction de déplacement d'une part, et
  - d'une distance non nulle selon un deuxième axe perpendiculaire à la direction de déplacement d'autre part.

Avantageusement, l'étape consistant à moduler peut comprendre la formation d'un motif comportant au moins deux (notamment trois) points d'impact adjacents s'étendant le long d'une ligne du motif, l'angle entre ladite ligne du motif et la direction de déplacement étant compris entre 10 et 80°, préférentiellement compris entre 15° et 40°, et encore plus préférentiellement entre 19° et 30°. Par ailleurs, l'étape de consistant à moduler peut comprendre la formation d'un motif ayant :

- un premier ensemble d'au moins deux (notamment trois) points d'impact disposés le long d'une première ligne du motif, et
- un deuxième ensemble d'au moins deux (notamment trois) autres points d'impact disposés le long d'une deuxième ligne du motif parallèle à la première ligne.

L'étape consistant à moduler peut également comprendre la formation d'un motif ayant au moins un autre ensemble de points d'impact disposés le long d'au moins une autre ligne du motif, parallèle aux première et deuxième lignes. L'étape consistant à moduler peut de plus comprendre la formation d'un motif dans lequel les points d'impact du deuxième ensemble sont décalés d'une distance non nulle par rapport aux points d'impact du premier ensemble. En variante, l'étape consistant à moduler peut comprendre la formation d'un motif dans lequel chaque point d'impact du deuxième ensemble est aligné avec un point d'impact respectif du premier ensemble selon une droite perpendiculaire à la direction de déplacement.

Avantageusement, l'étape consistant à moduler peut comprendre la formation d'un motif dans lequel la distance entre deux points d'impact adjacents est supérieure à 5μm préférentiellement supérieure à 10μm et encore plus préférentiellement comprise entre 10 et 15μm.

L'étape consistant à moduler peut en outre comprendre la formation d'un motif inscrit dans une surface dont le rapport entre la longueur et la largeur est compris entre 1 et 4, préférentiellement entre 1 et 2, et encore plus préférentiellement entre 1 et 1.5. Enfin, l'étape consistant à moduler peut également comprendre la formation d'un motif ayant un point d'impact central positionné au barycentre du motif.

*3. Principe de fonctionnement*

On va maintenant décrire le principe de fonctionnement de l'appareil de découpe illustré à la figure 1 en référence à la destruction d'un cristallin dans le cadre d'une opération de la cataracte. Il est bien évident que la présente invention ne se limite pas à l'opération d'une cataracte.

Dans une première étape, l'unité de commande 6 :

- transmet au système de mise en forme 3 un premier masque de phase associé à un premier motif de traitement,
- émet un signal de commande au système optique de focalisation 5 pour déplacer le plan de focalisation au niveau d'un premier plan de découpe profond dans l'oeil,
- active le déplacement du scanner optique de balayage 4 jusqu'à une position initiale de découpe. Le balayage se faisant en X, Y, le scanner est équipé d'un miroir, X, qui permet le balayage le long de chaque segment du chemin de déplacement du motif, et un autre miroir Y, qui permet une fois un segment complété, de changer de segment. Les miroirs X et Y fonctionnent donc alternativement l'un et l'autre.

Lorsque le système de focalisation 5 et le scanner optique 4 sont en position et que le masque de phase est chargé dans le système de mise en forme 3, l'unité de commande 6 active le laser femtoseconde 1. Celui-ci génère un faisceau L.A.S.E.R. 11 qui traverse le système de mise en forme 3. Le système de mise en forme 3 module la phase du faisceau L.A.S.E.R. Le faisceau L.A.S.E.R. modulé en phase 31 sort du système de mise en forme 3 et entre dans le scanner optique 4 qui dévie le faisceau de L.A.S.E.R. modulé 31. Le faisceau LA.S.E.R. modulé et dévié 41 entre dans le système optique de focalisation 5 qui focalise le faisceau dans le premier plan de découpe.

Chaque point d'impact 81 du motif 8 produit une bulle de cavitation. Le laser femtoseconde 1 continue d'émettre d'autres impulsions sous forme de faisceau LA.S.E.R. à une cadence déterminée. Entre chaque impulsion le miroir X a pivoté d'un certain angle, ce qui a pour conséquence de déplacer le motif 8 et de produire de nouvelles bulles de cavitation décalées par rapport aux précédentes, jusqu'à former une ligne. Ainsi, une première pluralité de bulles de cavitation constituant une ligne se forme dans le plan de découpe, ces bulles étant agencées conformément au motif de découpe 8. En faisant varier la vitesse de déplacement du miroir et/ou la cadence de génération des impulsions par le laser femtoseconde, il est possible de faire varier la distance entre deux motifs successifs.

Une fois cette pluralité de bulles formant une ligne complète, L'unité de commande 6 désactive la source L.A.S.E.R. 1, commande l'arrêt du pivotement du miroir X et commande le pivotement du miroir Y du scanner optique 4 jusqu'à la position suivante de découpe en fonction du pas de balayage du scanner optique 4, puis commande à nouveau le redémarrage du pivotement du miroir X dans le sens inverse. Lorsque le scanner optique 4 est en position et que le miroir X a atteint sa vitesse de consigne constante, l'unité de commande 6 active à nouveau le laser femtoseconde 1. Le faisceau L.A.S.E.R. 11 traverse le système de mise en forme 3, le scanner optique 4 et le système optique de focalisation 5. Une deuxième suite d'une pluralité de bulles de cavitation se forme dans le premier plan de découpe formant une nouvelle ligne parallèle à la précédente et juxtaposée.

Ces opérations sont répétées dans tout le premier plan de découpe.

Lorsque le scanner optique 4 a balayé toute la surface du premier plan de découpe, une première zone de découpe (dont la forme et les dimensions sont contrôlées par l'unité de commande 6) est générée.

L'unité de commande 6 désactive le laser femtoseconde 1 et commande :

- le déplacement en translation de la (ou des) lentille(s) du système optique de focalisation 5 pour déplacer le plan de focalisation 21 dans un deuxième plan de découpe,
- le déplacement en rotation du (ou des) miroir(s) du scanner optique 4 vers une position initiale de découpe du deuxième plan de découpe,
- le chargement éventuel par le système de mise en forme 3 d'un autre masque de phase pour modifier la disposition et/ou la taille des points d'impact du motif, etc.

L'unité de commande 6 répète les opérations de pilotage du laser femtoseconde 1, du système de mise en forme 3, du scanner optique de balayage 4 et du système de focalisation 5 dans le deuxième plan de découpe, et plus généralement dans les plans de découpe successifs.

A l'issue de ces différentes étapes, on obtient un empilement de plans de découpe correspondant au volume à détruire 23.

*4. Conclusions*

Ainsi, l'invention permet de disposer d'un outil de découpe efficace. Les dimensions des points d'impact du motif étant sensiblement égales (la forme, position et diamètre de chaque spot sont contrôlés dynamiquement par le masque de phase calculé et affiché sur le SLM et qui peut corriger les irrégularités), les bulles de cavitation qui dilacèrent les tissus biologiques découpés seront de tailles sensiblement égales. Ceci permet d'améliorer la qualité du résultat obtenu, avec un plan de découpe homogène, dans lequel les ponts tissulaires résiduels ont tous sensiblement la même taille et qui permettent une dissection par le praticien d'une qualité acceptable au regard de l'importance de la qualité de l'état de surface du tissu découpé lorsqu'il s'agit par exemple d'une cornée.

L'invention a été décrite pour des opérations de découpes d'une cornée dans le domaine de la chirurgie ophtalmologique, mais il est évident qu'elle peut être utilisée pour d'autre type d'opération en chirurgie ophtalmologique sans sortir du cadre de l'invention. Par exemple, l'invention trouve une application dans la chirurgie réfractive cornéenne, tel que le traitement des amétropies, notamment myopie, hypermétropie, astigmatisme, dans le traitement de la perte d'accommodation, notamment la presbytie.

L'invention trouve également une application dans le traitement de la cataracte avec incision de la cornée, découpe de la capsule antérieure du cristallin, et fragmentation du cristallin. Enfin, d'une manière plus générale, l'invention concerne toutes les applications cliniques ou expérimentales sur la cornée ou le cristallin d'un oeil humain ou animal.

D'une manière encore plus générale, l'invention concerne le domaine large de la chirurgie au L.A.S.E.R. et trouve une application avantageuse lorsqu'il s'agit de découper et plus particulièrement vaporiser des tissus mous humains ou animaux, à teneur en eau élevée.

Le lecteur aura compris que de nombreuses modifications peuvent être apportées à l'invention décrite précédemment sans sortir matériellement des nouveaux enseignements et des avantages décrits ici.

Par exemple, dans les différents modes de réalisation décrits précédemment, le système optique de focalisation disposé en aval du scanner optique de balayage était décrit comme comprenant un module unique permettant :

- d'une part de focaliser le faisceau L.A.S.E.R. modulé et dévié, et
- d'autre part de déplacer le plan de focalisation dans différents plans de découpe.

En variante, le système optique de focalisation peut être composé de deux modules distincts assurant chacun l'une de ces fonctions :

- un premier module - dit « module de positionnement en profondeur » - disposé en amont du scanner optique de balayage et permettant de déplacer le plan de focalisation dans différents plans de découpe.
- un deuxième module - dit « module concentrateur » disposé en aval du scanner optique de balayage et permettant de focaliser le faisceau L.A.S.E.R. modulé et dévié.

Egalement dans les différents modes de réalisation décrits précédemment, le système de mise en forme décrit était un SLM. En variante, le système de mise en forme pourrait être composé d'une pluralité de masques de phase, chaque masque de phase agissant sur la phase du faisceau L.A.S.E.R. pour répartir l'énergie du faisceau L.A.S.E.R. par modulation de phase selon un motif distinct. Chaque masque de phase peut par exemple être constitué d'une plaque (transparente au faisceau L.A.S.E.R.) d'épaisseur variable obtenue par gravure.

Dans ce cas, les masques de phase peuvent être fixés à un dispositif d'acheminement permettant de déplacer chaque masque de phase entre :

- une position active dans laquelle le masque de phase coupe le chemin optique du faisceau L.A.S.E.R.,
- une position inactive dans laquelle le masque de phase ne s'étend pas sur le chemin optique du faisceau L.A.S.E.R.

Le dispositif d'acheminement est par exemple constitué d'un support mobile en rotation autour d'un axe de rotation parallèle au chemin optique du faisceau L.A.S.E.R., le support mobile étant agencé de sorte à permettre le positionnement d'un masque de phase respectif sur le chemin optique du faisceau L.A.S.E.R. afin de moduler la phase de celui-ci. Toutefois cette solution nécessite l'introduction d'éléments mécaniques à l'appareil (dispositif d'acheminement) et ne constitue donc pas une solution préférée.

De plus, dans la description qui précède, l'unité de commande émettait un signal de commande au système de mise en forme (tel qu'un masque de phase dans le cas où le système de mise en forme est un modulateur spatial de lumière) pour répartir l'énergie du faisceau L.A.S.E.R. (par modulation de phase) en au moins deux points d'impact formant un motif dans son plan focal. En variante, l'unité de commande peut être programmée pour émettre plusieurs signaux de commande distincts permettant de générer des motifs différents les uns des autres. Ceci permet de modifier le profil d'intensité du faisceau L.A.S.E.R. selon différents motifs dans le plan de la découpe, par exemple pour améliorer la qualité de la découpe au niveau des contours de la surface découpée dans le plan de découpe.

Par conséquent, toutes les modifications de ce type sont destinées à être incorporées à l'intérieur de la portée des revendications jointes.

La présente demande de brevet européen est une demande divisionnaire de la demande de brevet européen numéro EP 17 714 828.5 qui revendiquait un appareil de découpe d'un tissu humain ou animal, telle qu'une cornée, ou un cristallin, ledit appareil incluant un laser femtoseconde apte à émettre un faisceau L.A.S.E.R. sous forme d'impulsions et un dispositif de traitement pour produire un motif composé d'au moins deux points d'impact dans un plan de focalisation à partir du faisceau L.A.S.E.R. généré par le laser femtoseconde, le dispositif de traitement étant disposé en aval dudit laser femtoseconde, remarquable en ce que le dispositif de traitement comprend : un système optique de focalisation pour focaliser le faisceau L.A.S.E.R. dans un plan de découpe, et une unité de commande apte à piloter le déplacement du système optique de focalisation le long d'un chemin optique du faisceau L.A.S.E.R. pour déplacer le plan de focalisation en au moins trois plans de découpe respectifs de sorte à former un empilement de surfaces de découpe du tissu.

On entend, dans le cadre de la présente invention, par *« point d'impact »* une zone du faisceau L.A.S.E.R. comprise dans son plan focal dans laquelle l'intensité dudit faisceau L.A.S.E.R. est suffisante pour générer une bulle de cavitation dans un tissu.

On entend, dans le cadre de la présente invention, par *« points d'impact adjacents »,* deux points d'impact disposés en regard l'un de l'autre et non séparés par un autre point d'impact. On entend par *« points d'impact voisins »* deux points d'un groupe de points adjacents entre lesquels la distance est minimale.

On entend, dans le cadre de la présente invention, par *« motif »* une pluralité de points d'impact L.A.S.E.R. générés simultanément dans un plan de focalisation d'un faisceau L.A.S.E.R. mis en forme - c'est-à-dire modulé en phase pour répartir son énergie en plusieurs spots distincts dans le plan de focalisation correspondant au plan de découpe du dispositif.

Ainsi, l'invention permet de modifier le profil d'intensité du faisceau L.A.S.E.R. dans le plan de découpe, d'une manière à pouvoir améliorer la qualité ou bien la vitesse de la découpe en fonction du profil choisi. Cette modification de profil d'intensité est obtenue par modulation de la phase du faisceau L.A.S.E.R.

La modulation optique de phase est réalisée au moyen d'un masque de phase. L'énergie du faisceau L.A.S.E.R. incident est conservée après modulation, et la mise en forme du faisceau est réalisée en agissant sur son front d'onde. La phase d'une onde électromagnétique représente la situation instantanée de l'amplitude d'une onde électromagnétique. La phase dépend aussi bien du temps que de l'espace. Dans le cas de la mise en forme spatiale d'un faisceau L.A.S.E.R., seules les variations dans l'espace de la phase sont considérées.

Le front d'onde est défini comme la surface des points d'un faisceau possédant une phase équivalente (i.e. la surface constituée des points dont les temps de parcours depuis la source ayant émis le faisceau sont égaux). La modification de la phase spatiale d'un faisceau passe donc par la modification de son front d'onde.

Cette technique permet de réaliser l'opération de découpe d'une manière plus rapide et plus efficace car elle met en oeuvre plusieurs spots L.A.S.E.R. réalisant chacun une découpe et selon un profil contrôlé.

Dans le cadre de la présente invention, la modulation de phase du front d'onde permet de générer un unique faisceau L.A.S.E.R. modulé qui forme plusieurs points d'impact seulement dans le plan de découpe. Ainsi, le faisceau L.A.S.E.R. modulé est unique tout au long du chemin de propagation. La modulation de phase du front d'onde permet de retarder ou d'avancer la phase des différents points de la surface du faisceau par rapport au front d'onde initial afin que chacun de ces points réalisent une interférence constructive en N points distincts dans le plan focal d'une lentille. Cette redistribution d'énergie en une pluralité de points d'impact n'a lieu que dans un seul plan (i.e. le plan de focalisation) et pas tout au long du chemin de propagation du faisceau L.A.S.E.R. modulé. Au contraire, le document US 2010/0133246 propose d'utiliser un système optique basé sur la phase et permettant de subdiviser un faisceau primaire en une pluralité de faisceaux secondaires ayant des angles de propagation différents.

La technique de modulation selon l'invention (par génération d'un unique faisceau L.A.S.E.R. modulé) permet de limiter les risques de dégradation de la qualité de la surface découpée. En effet, si une portion de l'unique faisceau L.A.S.E.R. modulé est perdue le long du chemin de propagation du faisceau, les intensités de tous les points d'impact du motif seront atténuées en même temps (conservation de l'homogénéité entre les différents points d'impact du motif) mais aucun point d'impact ne disparaitra dans le plan de découpe. Au contraire avec la technique de subdivision de faisceau proposée dans US 2010/0133246, si une portion de la pluralité de faisceaux secondaires est perdue le long du chemin de propagation, alors certains points d'impact du motif (correspondant aux points d'impact générés par les faisceaux secondaires perdus) seront absents dans le plan de découpe, ce qui dégrade sensiblement la qualité de la découpe effectuée.

Des aspects préférés mais non limitatifs de l'appareil de découpe selon EP 17 714 828.5 étaient les suivants :

- l'unité de de commande peut être apte à piloter le déplacement du système optique de focalisation pour déplacer le plan de focalisation entre une position initiale et une position finale dans cet ordre, la position finale étant plus proche du laser femtoseconde que la position initiale ;
- l'appareil peut comprendre en outre un système de mise en forme positionné entre le laser femtoseconde et le système optique de focalisation, pour moduler la phase du front d'onde du faisceau L.A.S.E.R. de sorte à obtenir un faisceau L.A.S.E.R. modulé en phase selon une consigne de modulation calculée pour répartir l'énergie du faisceau L.A.S.E.R. en au moins deux points d'impact formant le motif dans son plan focal ;
- la longueur du chemin optique entre le système de mise en forme et le système optique de focalisation peut être inférieure à 2 mètres, de préférence inférieure à 1 mètre ;
- l'unité de commande peut être programmée pour piloter le système de mise en forme de sorte à faire varier la forme du motif entre deux plans de découpe respectifs ;
- l'unité de commande peut être programmée pour piloter le système de mise en forme, ladite unité de commande étant adaptée pour émettre au moins des premier et deuxième signaux de commande entre deux plans de découpe respectifs (ou dans un même plan de découpe) :

  - le premier signal de commande induisant la modulation de la phase du front d'onde du faisceau L.A.S.E.R. selon une première consigne de modulation calculée pour répartir l'énergie du faisceau L.A.S.E.R. en une pluralité de premiers points d'impact dans le plan focal du système de mise en forme, les premiers points d'impact constituant un premier motif,
  - - le deuxième signal de commande induisant la modulation de la phase du front d'onde du faisceau L.A.S.E.R. selon une deuxième consigne de modulation calculée pour répartir l'énergie du faisceau L.A.S.E.R. en une pluralité de deuxièmes points d'impact dans le plan focal du système de mise en forme, les deuxièmes points d'impact constituant un deuxième motif différent du premier motif ;

- l'appareil peut comprendre en outre un scanner optique de balayage disposé en aval du laser femtoseconde, pour déplacer le motif dans le plan de découpe en une pluralité de positions selon une direction de déplacement ;
- l'unité de commande peut être programmée pour piloter le scanner optique de balayage de sorte à faire varier l'aire découpée dans le plan de focalisation entre deux plans de découpe successifs ;
- l'unité de commande peut être programmée pour piloter le scanner optique de balayage de sorte à faire varier la forme de la zone découpée dans le plan de focalisation entre deux plans de découpe successifs ;
- l'unité de commande peut être programmée pour piloter le scanner optique de sorte à faire varier un pas de balayage du scanner optique entre deux plans de découpe successifs ;
- l'unité de commande peut être apte à piloter le déplacement du système optique de focalisation de sorte que la distance entre deux plans successifs soit comprise entre 2 $\mu$m et 500 $\mu$m
- l'appareil peut comprendre en outre un filtre disposé en aval du système de mise en forme pour bloquer une énergie parasite générée au centre du système de mise en forme ;
- le filtre peut comprendre une plaque incluant : une zone opaque au rayonnement L.A.S.E.R. disposée au centre de la plaque, et une zone transparente au rayonnement L.A.S.E.R. s'étendant à la périphérie de la zone opaque.
- le système de mise en forme peut consister en un ensemble de masques de phase, chaque masque agissant sur la phase du faisceau L.A.S.E.R. pour répartir l'énergie du faisceau L.A.S.E.R. par modulation de phase selon un motif distinct, les masques étant fixés à un dispositif d'acheminement, l'unité de commande étant programmée pour piloter le dispositif d'acheminement (par émission d'un ou plusieurs signaux de commande) afin de déplacer chaque masque entre : une position active dans laquelle le masque coupe le chemin optique du faisceau L.A.S.E.R., et une position inactive dans laquelle le masque ne s'étend pas sur le chemin optique du faisceau L.A.S.E.R. ;
- le système de mise en forme peut en variante consister en un modulateur spatial de lumière, l'unité de commande étant programmée pour piloter le modulateur spatial de lumière par émission d'au moins un signal de commande induisant la modulation de la phase du front d'onde du faisceau L.A.S.E.R. selon une consigne de modulation calculée pour répartir l'énergie du faisceau L.A.S.E.R. en une pluralité de points d'impact dans le plan focal du système de mise en forme,
- la consigne de modulation peut être un masque de phase calculé en utilisant un algorithme itératif basé sur la transformée de Fourier.

## Revendications

**1.** Appareil de découpe d'un tissu humain ou animal, telle qu'une cornée, ou un cristallin, ledit appareil incluant un laser femtoseconde (1) apte à émettre un faisceau L.A.S.E.R. (11) sous forme d'impulsions et un dispositif de traitement pour produire un motif (8) dans un plan de focalisation (21) à partir du faisceau L.A.S.E.R. généré par le laser femtoseconde, le dispositif de traitement étant disposé en aval dudit laser femtoseconde, le dispositif de traitement comprenant:

- un modulateur spatial de lumière (SLM) entre la source (1) et le plan de focalisation (21), ledit modulateur spatial de lumière (SLM) étant programmé à l'aide d'au moins un masque de phase pour moduler la phase du front d'onde du faisceau L.A.S.E.R. (11) issu du laser femtoseconde (1) de sorte à obtenir un faisceau L.A.S.E.R. modulé,
- un système optique de focalisation (5) pour focaliser le faisceau L.A.S.E.R. modulé dans un plan de découpe, et
- une unité de commande (6) apte à piloter le déplacement du système optique de focalisation le long d'un chemin optique du faisceau L.A.S.E.R. pour déplacer le plan de focalisation (21) en au moins trois plans de découpe respectifs de sorte à former un empilement de surfaces de découpe du tissu.

**2.** Appareil de découpe selon la revendication 1, ***dans lequel*** l'unité de de commande est apte à piloter le déplacement du système optique de focalisation pour déplacer le plan de focalisation entre une position initiale et une position finale dans cet ordre, la position finale étant plus proche du laser femtoseconde que la position initiale.

**3.** Appareil de découpe selon l'une quelconque des revendications 1 ou 2, *dans lequel* chaque masque de phase est une image bidimensionnelle dont chaque point est associé à un pixel respectif du modulateur spatial de lumière (SLM), ladite image bidimensionnelle déterminant comment la phase du faisceau doit être modifiée pour obtenir une répartition d'amplitude donnée dans le plan de focalisation.

**4.** Appareil de découpe selon l'une quelconque des revendications 1 à 3, ***dans lequel*** le modulateur spatial de lumière (SLM) est positionné entre le laser femtoseconde et le système optique de focalisation, au moins un masque de phase étant calculé pour répartir l'énergie du faisceau L.A.S.E.R. modulé en au moins deux points d'impact formant le motif dans le plan de focalisation.

**5.** Appareil de découpe selon la revendication 4, dans lequel ledit et au moins un masque de phase est calculé en utilisant un algorithme itératif basé sur la transformée de Fourier de sorte à obtenir un unique faisceau L.A.S.E.R. modulé en phase.

**6.** Appareil de découpe selon l'une quelconque des revendications 1 à 5, *dans lequel* le modulateur spatial de lumière (SLM) est positionné entre le laser femtoseconde et le système optique de focalisation, au moins un masque de phase étant calculé pour modifier la forme géométrique d'un point d'impact du faisceau L.A.S.E.R. modulé, de sorte que :

- la forme géométrique, dans le plan de focalisation, d'un point d'impact du faisceau L.A.S.E.R. issu du laser femtoseconde est différente de
- la forme géométrique, dans le plan de focalisation, d'un point d'impact du faisceau L.A.S.E.R. modulé.

**7.** Appareil selon l'une quelconque des revendications 1 à 6, ***dans lequel*** l'unité de commande est programmée pour piloter le modulateur spatial de lumière (SLM) de sorte à faire varier la forme du motif entre deux plans de découpe respectifs.

**8.** Appareil selon la revendication 7, ***dans lequel*** l'unité de commande est programmée pour piloter le modulateur spatial de lumière (SLM), ladite unité de commande (6) étant adaptée pour émettre au moins des premier et deuxième signaux de commande entre deux plans de découpe respectifs :

- le premier signal de commande induisant la modulation de la phase du front d'onde du faisceau L.A.S.E.R. selon un premier masque de phase calculé pour répartir l'énergie du faisceau L.A.S.E.R. modulé en une pluralité de premiers points d'impact dans le plan de focalisation, les premiers points d'impact constituant un premier motif,
- le deuxième signal de commande induisant la modulation de la phase du front d'onde du faisceau L.A.S.E.R. selon un deuxième masque de phase calculé pour répartir l'énergie du faisceau L.A.S.E.R. modulé en une pluralité de deuxièmes points d'impact dans le plan de focalisation, les deuxièmes points d'impact constituant un deuxième motif différent du premier motif.

**9.** Appareil de découpe selon l'une quelconque des revendications 1 à 8, ***lequel*** comprend en outre :

- un scanner optique de balayage disposé en aval du laser femtoseconde, pour déplacer le motif dans le plan de découpe en une pluralité de positions selon une direction de déplacement.

10. Appareil selon la revendication 9, ***dans lequel*** l'unité de commande est programmée pour piloter le scanner optique de balayage de sorte à faire varier l'aire découpée dans le plan de focalisation entre deux plans de découpe successifs.

11. Appareil selon l'une quelconque des revendications 9 ou 10, ***dans lequel*** l'unité de commande est programmée pour piloter le scanner optique de balayage de sorte à faire varier la forme de la zone découpée dans le plan de focalisation entre deux plans de découpe successifs.

12. Appareil selon l'une quelconque des revendications 9 à 11, ***dans lequel*** l'unité de commande est programmée pour piloter le scanner optique de sorte à faire varier un pas de balayage du scanner optique entre deux plans de découpe successifs.

13. Appareil de découpe selon l'une quelconque des revendications 1 à 12, ***dans lequel*** l'unité de commande est apte à piloter le déplacement du système optique de focalisation de sorte que la distance entre deux plans successifs soit comprise entre 2 $\mu$m et 500 $\mu$m.

14. Appareil selon l'une quelconque des revendications 1 à 13, ***lequel*** comprend en outre un filtre disposé en aval du modulateur spatial de lumière (SLM) pour bloquer une énergie parasite générée au centre du modulateur spatial de lumière (SLM).

15. Appareil selon la revendication 14, ***dans lequel*** le filtre comprend une plaque incluant :

- une zone opaque au rayonnement L.A.S.E.R. disposée au centre de la plaque, et
- une zone transparente au rayonnement L.A.S.E.R. s'étendant à la périphérie de la zone opaque.

**Patentansprüche**

1. Vorrichtung zum Schneiden eines menschlichen oder tierischen Gewebes, wie eine Hornhaut oder ein Linsenkörper, wobei die Vorrichtung einen Femtosekundenlaser (1), der dazu fähig ist, einen L.A.S.E.R.-Strahl (11) in der Form von Impulsen abzugeben, und ein Behandlungsgerät zum Produzieren eines Musters (8) in einer Fokussierebene (21) von dem L.A.S.E.R.-Strahl, der von dem Femtosekundenlaser erzeugt wird, beinhaltet, wobei das Behandlungsgerät hinter dem Femtosekundenlaser angeordnet ist, wobei das Behandlungsgerät umfasst:

- einen räumlichen Lichtmodulator (SLM) zwischen der Quelle (1) und der Fokussierebene (21), wobei der räumliche Lichtmodulator (SLM) mithilfe mindestens einer Phasenmaske zum Modulieren der Phase der Wellenfront des L.A.S.E.R.-Strahls (11), der von dem Femtosekundenlaser (1) erzeugt wird, um einen modulierten L.A.S.E.R.-Strahl zu erhalten,
- ein optisches Fokussiersystem (5) zum Fokussieren des modulierten L.A.S.E.R.-Strahls in einer Schneidebene, und
- eine Steuereinheit (6), die dazu fähig ist, die Verlagerung des optischen Fokussiersystems entlang einem optischen Weg des L.A.S.E.R.-Strahls zu steuern, um die Fokussierebene (21) in mindestens drei jeweiligen Schneidebenen zu verlagern, um einen Stapel von Schneidflächen des Gewebes zu bilden.

2. Schneidvorrichtung nach Anspruch 1, wobei die Steuereinheit dazu fähig ist, die Verlagerung des optischen Fokussiersystems zu steuern, um die Fokussierebene zwischen einer Anfangsposition und einer Endposition in dieser Reihenfolge zu verlagern, wobei die Endposition dem Femtosekundenlaser näher ist als die Anfangsposition.

3. Schneidvorrichtung nach einem der Ansprüche 1 oder 2, wobei jede Phasenmaske ein zweidimensionales Bild ist, von dem jeder Punkt mit einem jeweiligen Pixel des räumlichen Lichtmodulators (SLM) assoziiert ist, wobei das zweidimensionale Bild bestimmt, wie die Phase des Strahls modifiziert werden muss, um eine gegebene Amplitudenverteilung in der Fokussierebene zu erhalten.

4. Schneidvorrichtung nach einem der Ansprüche 1 bis 3, wobei der räumliche Lichtmodulator (SLM) zwischen dem Femtosekundenlaser und dem optischen Fokussiersystem positioniert ist, wobei mindestens eine Phasenmaske dazu berechnet ist, die Energie des modulierten L.A.S.E.R.-Strahls in mindestens zwei Auftreffpunkte, die das Muster in der Fokussierebene bilden, aufzuteilen.

5. Schneidvorrichtung nach Anspruch 4, wobei die mindestens eine Phasenmaske unter Verwendung eines iterativen Algorithmus auf der Basis der Fourier-Transformation berechnet wird, um einen einzigen phasenmodulierten L.A.S.E.R.-Strahl zu erhalten.

6. Schneidvorrichtung nach einem der Ansprüche 1 bis 5, wobei der räumliche Lichtmodulator (SLM) zwischen dem Femtosekundenlaser und dem optischen Fokussiersystem positioniert ist, wobei mindestens eine Phasenmaske dazu berechnet ist, die geometrische Form eines Auftreffpunkts des modulierten L.A.S.E.R.-Strahls zu modifizieren, so dass:

   - die geometrische Form eines Auftreffpunkts des L.A.S.E.R.-Strahls, der aus dem Femtosekundenlaser hervorgeht, in der Fokussierebene sich von
   - der geometrischen Form eines Auftreffpunkts des modulierten L.A.S.E.R.-Strahls in der Fokussierebene unterscheidet.

7. Vorrichtung nach einem der Ansprüche 1 bis 6, wobei die Steuereinheit dazu programmiert ist, den räumlichen Lichtmodulator (SLM) zu steuern, um die Form des Musters zwischen zwei jeweiligen Schneidebenen zu variieren.

8. Vorrichtung nach Anspruch 7, wobei die Steuereinheit dazu programmiert ist, den räumlichen Lichtmodulator (SLM) zu steuern, wobei die Steuereinheit (6) dazu eingerichtet ist, mindestens erste und zweite Steuersignale zwischen zwei jeweiligen Schneidebenen abzugeben:

   - das erste Steuersignal induziert die Modulation der Phase der Wellenfront des L.A.S.E.R.-Strahls gemäß einer ersten Phasenmaske, die dazu berechnet ist, die Energie des modulierten L.A.S.E.R.-Strahls auf eine Vielzahl von ersten Auftreffpunkten in der Fokussierebene aufzuteilen, wobei die ersten Auftreffpunkte ein erstes Muster bilden,
   - das zweite Steuersignal induziert die Modulation der Phase der Wellenfront des L.A.S.E.R.-Strahls gemäß einer zweiten Phasenmaske, die dazu berechnet ist, die Energie des modulierten L.A.S.E.R.-Strahls auf eine Vielzahl von zweiten Auftreffpunkten in der Fokussierebene aufzuteilen, wobei die zweiten Auftreffpunkte ein zweites Muster bilden, das sich von dem ersten Muster unterscheidet.

9. Schneidvorrichtung nach einem der Ansprüche 1 bis 8, die ferner umfasst:

   - einen optischen Abtastscanner, der hinter dem Femtosekundenlaser angeordnet ist, um das Muster in der Schneidebene in eine Vielzahl von Positionen gemäß einer Verlagerungsrichtung zu verlagern.

10. Vorrichtung nach Anspruch 9, wobei die Steuereinheit dazu programmiert ist, den optischen Abtastscanner zu steuern, um die geschnittene Fläche in der Fokussierebene zwischen zwei aufeinanderfolgenden Schneidebenen zu variieren.

11. Vorrichtung nach einem der Ansprüche 9 oder 10, wobei die Steuereinheit dazu programmiert ist, den optischen Abtastscanner zu steuern, um die Form der geschnittenen Zone in der Fokussierebene zwischen zwei aufeinanderfolgenden Schneidebenen zu variieren.

12. Vorrichtung nach einem der Ansprüche 9 bis 11, wobei die Steuereinheit dazu programmiert ist, den optischen Scanner zu steuern, um eine Abtastschrittweite des optischen Scanners zwischen zwei aufeinanderfolgenden Schneidebenen zu variieren.

13. Schneidvorrichtung nach einem der Ansprüche 1 bis 12, wobei die Steuereinheit dazu fähig ist, die Verlagerung des optischen Fokussiersystems zu steuern, so dass der Abstand zwischen zwei aufeinanderfolgenden Ebenen zwischen 2 μm und 500 μm liegt.

14. Vorrichtung nach einem der Ansprüche 1 bis 13, die ferner ein Filter umfasst, das hinter dem räumlichen Lichtmodulator (SLM) angeordnet ist, um eine Störenergie, die in der Mitte des räumlichen Lichtmodulators (SLM) erzeugt wird, zu blockieren.

15. Vorrichtung nach Anspruch 14, wobei das Filter eine Platte umfasst, beinhaltend:

- eine für L.A.S.E.R.-Strahlung undurchlässige Zone, die in der Mitte der Platte angeordnet ist, und
- eine für L.A.S.E.R.-Strahlung durchlässige Zone, die sich vom Rand der undurchlässigen Zone erstreckt.

## Claims

1. An apparatus for cutting human or animal tissue, such as a cornea, or a lens, said apparatus including a femtosecond laser (1) capable of emitting a laser beam (11) in the form of pulses and a processing device for producing a pattern (8) in a focusing plane (21) from the laser beam generated by the femtosecond laser, the processing device being positioned downstream of said femtosecond laser, the processing device comprising:

   - a spatial light modulator (SLM) between the source (1) and the focusing plane (21), said spatial light modulator (SLM) being programmed using at least one phase mask for modulating the phase of the wavefront of the laser beam (11) from the femtosecond laser (1) so as to obtain a modulated laser beam,
   - a focusing optical system (5) for focusing the modulated laser beam in a cutting plane, and
   - a control unit (6) capable of controlling the movement of the focusing optical system along an optical path of the laser beam to displace the focusing plane (21) into at least three respective cutting planes so as to form a stack of tissue cutting surfaces.

2. The cutting apparatus as claimed in claim 1, wherein the control unit is capable of controlling the movement of the focusing optical system to move the focusing plane between an initial position and a final position in that order, the final position being closer to the femtosecond laser than the initial position.

3. The cutting apparatus as claimed in any one of claims 1 or 2, wherein each phase mask is a two-dimensional image, each point of which is associated with a respective pixel of the spatial light modulator (SLM), said two-dimensional image determining how the phase of the beam must be modified to obtain a given amplitude distribution in the focusing plane.

4. The cutting apparatus as claimed in any one of claims 1 to 3, wherein the spatial light modulator (SLM) is positioned between the femtosecond laser and the focusing optical system, at least one phase mask being calculated to distribute the energy of the modulated laser beam into at least two impact points forming the pattern in the focusing plane.

5. The cutting apparatus as claimed in claim 4, wherein said at least one phase mask is calculated using a Fourier transform-based iterative algorithm so as to obtain a single phase-modulated laser beam.

6. The cutting apparatus as claimed in any one of claims 1 to 5, wherein the spatial light modulator (SLM) is positioned between the femtosecond laser and the focusing optical system, at least one phase mask being calculated to modify the geometric shape of an impact point of the modulated laser beam, so that:

   - the geometric shape, in the focusing plane, of an impact point of the laser beam from the femtosecond laser is different from
   - the geometric shape, in the focusing plane, of an impact point of the modulated laser beam.

7. The apparatus as claimed in any one of claims 1 to 6, wherein the control unit is programmed to control the spatial light modulator (SLM) so as to vary the shape of the pattern between two respective cutting planes.

8. The apparatus as claimed in claim 7, wherein the control unit is programmed to control the spatial light modulator (SLM), said control unit (6) being adapted to transmit at least first and second control signals between two respective cutting planes:

   - the first control signal causing modulation of the phase of the wavefront of the laser beam according to a first phase mask calculated to distribute the energy of the modulated laser beam into a plurality of first impact points in the focusing plane, the first impact points constituting a first pattern,
   - the second control signal causing modulation of the phase of the wavefront of the laser beam according to a second phase mask calculated to distribute the energy of the modulated laser beam into a plurality of second impact points in the focusing plane, the second impact points constituting a second pattern different from the first pattern.

**9.** The cutting apparatus as claimed in any one of claims 1 to 8, which further comprises:

- a sweeping optical scanner positioned downstream of the femtosecond laser, for moving the pattern in the cutting plane to a plurality of positions along a direction of movement.

**10.** The apparatus as claimed in claim 9, wherein the control unit is programmed to control the sweeping optical scanner so as to vary the area cut in the focusing plane between two successive cutting planes.

**11.** The apparatus as claimed in any one of claims 9 or 10, wherein the control unit is programmed to control the sweeping optical scanner so as to vary the shape of the zone cut in the focusing plane between two successive cutting planes.

**12.** The apparatus as claimed in any one of claims 9 to 11, wherein the control unit is programmed to control the optical scanner so as to vary a sweeping pitch of the optical scanner between two successive cutting planes.

**13.** The cutting apparatus as claimed in any one of claims 1 to 12, wherein the control unit is capable of controlling the movement of the focusing optical system so that the distance between two successive planes is between 2 um and 500 $\mu$m.

**14.** The apparatus as claimed in any one of claims 1 to 13, which further comprises a filter positioned downstream of the spatial light modulator (SLM) to block parasitic energy generated at the center of the spatial light modulator (SLM).

**15.** The apparatus as claimed in claim 14, wherein the filter comprises a plate including:

- a zone opaque to laser radiation positioned at the center of the plate, and
- a zone transparent to laser radiation extending to the periphery of the opaque zone.

4
31
3
11
1
41
5
51
21
2
6

FIG. 1

1
11
5
21
33a
32a
7
71
5
33b
32b
33c
32c
1
11
3
31
5
21
33d
32d
33e
32e

FIG. 2

42a
42b
42c
42
8
81
dist
éc
81
81
43a
43b
43c
23
2
21
22a
22b
22d
22c
22e

FIG. 3

FIG. 4

8
81d
83
81e
81f
82
D
81a
81b
81c

FIG. 5

82
83
8
81a
81d
81b
81e
D
81c
81f

FIG. 6

81a
8
82
81b
81c
e
α
d
D
8
81d
81c
83
α
82
D
81b
81a
8
83
81d
81e
81f
82
D
81a
81b
81c

FIG. 7

FIG. 8

FIG. 9

100

D

101

FIG. 10

FIG. 11a

FIG. 11b

FIG. 11c

D * cos(30°) * 2
D * cos(30°) * 2
°30
60°
90°


FIG. 12

FIG. 13
D
C
A
B
B
FIG. 14
A = D * cos(30°) * 4
B = √((2,5D)²+(cos(30°) * D)²)
C = √((4,5D)²+(cos(30°) * 5D)²)
C
A
B
B
β = 16,1°
FIG. 15
A = D * cos(30°) * 4
B = √((2,5D)²+(cos(30°) * D)²)
C = √((4,5D)²+(cos(30°) * 5D)²)
α = 19,1°
β = 16,1°
D X 2,5
α = 19,1°
90°
70,9°
D X Cos(30°)
√((2,5D)²+(cos(30°) * D)²)

FIG. 16
D
C
A
A
A
A
$A = \sqrt{(2,5D)^2 + (\cos(30°) * D)^2}$
$C = \sqrt{(5,5D)^2 + (\cos(30°) * 5D)^2}$
FIG. 17
C
A
A
A
$A = \sqrt{(2,5D)^2 + (\cos(30°) * D)^2}$
$C = \sqrt{(5,5D)^2 + (\cos(30°) * 5D)^2}$
α = 19,1°
β = 16,1°
β = 16,1°
D X 2,5
α = 19,1°
90°
70,9°
D X Cos(30°)
$\sqrt{(2,5D)^2 + (\cos(30°) * D)^2}$
FIG. 18

100
D
101
FIG. 19
FIG. 20

C
A
E
B
$A = \sqrt{8D^2}$
$B = \sqrt{5D^2}$
$C = \sqrt{41D^2}$
$E = 3D$
$\alpha = 26{,}56°$
D * 2
α = 26,56°
90°
D
63,44°
$\sqrt{5D^2}$
FIG. 21

5µm
5µm
90°
102
103

FIG. 22

105
106
104
D

FIG. 23

81
81

81
81

FIG. 24

ZNT
81

FIG. 25

109
108
107
105

FIG. 26

FIG. 27

81P
81B
81P

FIG. 28

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2010133246 A
- EP 1790383 A
- US 2016067095 A
- US 20100133246 A
- US 20150164689 A
- EP 17714828 A